# EUROPEAN PATENT SPECIFICATION

(11) **EP 0 624 163 B1**
(45) Date of publication and mention of the grant of the patent: **25.03.1998**
(21) Application number: 93903212.4
(22) Date of filing: 20.01.1993
(51) Int. Cl.: C07K 14/585, A61K 38/23

(54) **CYCLIC CALCITONIN DERIVATIVES AND METHOD FOR THEIR PRODUCTION**
Zyklische Calcitonin derivate und ihr Herstellungsverfahren
DERIVES DE CALCITONINE CYCLIQUES ET LEUR PROCEDE DE PRODUCTION

(30) Priority: 24.01.1992 GB 9201819
(43) Date of publication of application: 17.11.1994
(73) Proprietor: Smithkline Beecham Farmaceutici S.p.A., 20021 Baranzate di Bollate (MI) (IT)
(72) Inventor: MENA, Renzo SmithKline Beecham Farmaceutici S.p.A., I-20021 Milan (IT); BRUGNOLOTTI, Manuela, SmithKline Beecham Farm. SpA, I-20021 Milan (IT); FARINA, Carlo, SmithKline Beecham Farmaceutici SpA, I-20021 Milan (IT); PINZA, Mario SmithKline Beecham Farmaceutici S.p.A, I-20021 Milan (IT)
(74) Representative: Waters, David Martin, Dr.
(86) International application number: EP9300130
(87) International publication number: WO9315106

(56) References cited:
- EP-A- 0 181 121
- JOURNAL OF ORGANIC CHEMISTRY vol. 54, no. 17, 18 August 1989, EASTON US pages 4224 - 4228 D. WERNIC ET AL. 'Enantiospecific Synthesis of L-.alpha.-Aminosuberic Acid. Synthetic Applications in Preparation of Atrial Natriuretic Factor Analogues' cited in the application
- MONATSHEFTE FUR CHEMIE vol. 89, no. 3, 19 August 1958, WIEN AT pages 377 - 390 K. SCHL GL 'Acetylen-Aminos uren'

## Description

The present invention relates to novel compounds, processes for preparing them, pharmaceutical compositions containing them and their use in therapy. More particularly this invention concerns novel calcitonin derivatives.

The calcitonins are a class of pharmacologically active peptides, of both natural and synthetic origin, which contain approximately thirty two amino acids, and which have the ability to regulate serum calcium levels. Examples of naturally occuring calcitonins include rat, salmon, eel, chicken, ox, pig, sheep and human calcitonins. Examples of synthetically modified calcitonins include 1,7-dicarba-calcitonins such as eel 1,7-dicarba-calcitonin (elcatonin), salmon 1,7-dicarba-calcitonin and human 1,7-dicarba-calcitonin. Various calcitonins, including e.g. natural human, salmon and eel calcitonins and the synthetic eel calcitonin analogue elcatonin are now commercially available and commonly employed, e.g. in the treatment of Paget's disease, Sudeck's disease and osteoporosis. J. Org. Chem. (1989), 54 (17), 4224-4228 describes the synthesis of certain atrial natriuretic factor analogues having *inter alia* a dehydro-L-α-aminosuberic acid bridge.

We have now found a group of novel, synthetically modified calcitonins, having interesting hypocalcaemic activity.

In a first aspect therefore the present invention provides compounds of formula (I): 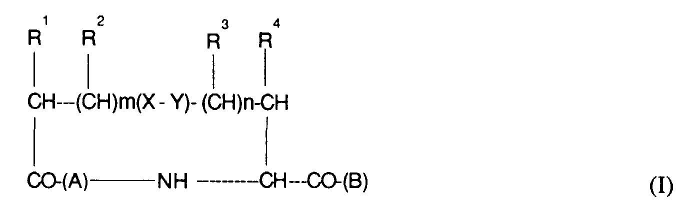 wherein:
- (X-Y): represents CH=CH or C≡C;
- A: represents represents fragments 2-6 of a naturally occurring calcitonin, or a modified variant thereof which is a peptide residue: -A2-A3-Leu-Ser-Thr wherein A₂ represents Ser, Gly or Ala and A₃ represents Asn or Ser;
- B: represents fragments 8-32 of a naturally occurring calcitonin, or a modified variant thereof, which modified variant has the formula: 
-Val-Leu-Gly-Lys-Leu-Ser-Gln-Glu-Leu-His-Lys-C-Pro-Arg-Thr-D-Gly-E-Gly-
Thr-Pro-NH2
 in which
C represents -Leu-Gln-Thr-Tyr; Gln-Thr-Tyr; or a bond;
D represents -Asn-Thr; -Asp-Val; -Ala-Thr or -Ala-Val; and
E represents Ser or Ala;
- m and n: each independently represent 0, 1 or 2; and
- R¹, R², R³, and R⁴: independently represent hydrogen or a substituent group e.g. a C₁₋₄ alkyl group.

In the compounds of Formula (I) modified variants of fragments A and B, refer to such modified fragments as are known in the art, and include fragments wherein one or more amino acids in the naturally occurring sequence have been replaced or deleted.

Preferably fragment (A) represents fragments 2-6 corresponding to those found in naturally occurring pig, ox, sheep, human, rat, eel, salmon or chicken calcitonins for example: 
-Ser-Asn-Leu-Ser-Thr (pig, ox, sheep, eel, salmon)
-Gly-Asn-Leu-Ser-Thr (human, rat)
 or

-Ala-Ser-Leu-Ser-Thr (chicken)

Most preferably (A) represents: -Ser-Asn-Leu-Ser-Thr- Fragment (B) preferably represents fragments 8-32 corresponding to those found in naturally occurring calcitonins, for example:

-Met-Leu-Gly-Thr-Tyr-Thr-Gln-Asp-Phe-Asn-Lys-Phe-His-Thr-Phe-Pro-Gln-Thr-Ala-Ile-
Gly-Val-Gly-Ala-Pro-NH2; (human)

-Met-Leu-Gly-Thr-Tyr-Thr-Gln-Asp-Leu-Asn-Lys-Phe-His-Thr-Phe-Pro-Gln-Thr-Ser-Ile-
Gly-Val-Gly-Ala-Pro-NH2; (rat)

-Val-Leu-Gly-Lys-Leu-Ser-Gln-Glu-Leu-His-Lys-Leu-Gln-Thr-Tyr-Pro-Arg-Thr-Asn-Thr-
Gly-Ser-Gly-Thr-Pro-NH2; (salmon)

-Val-Leu-Gly-Lys-Leu-Ser-Gln-Glu-Leu-His-Lys-Leu-Gln-Thr-Tyr-Pro-Arg-Thr-Asp-Val-
Gly-Ala-Gly-Thr-Pro-NH2; (eel, chicken)

-Val-Leu-Ser-Ala-Tyr-Trp-Lys-Asp-Leu-Asn-Asn-Tyr-His-Arg-Phe-Ser-Gly-Met-Gly-Phe-
Gly-Pro-Glu-Thr-Pro-NH2; (ox)

-Val-Leu-Ser-Ala-Tyr-Trp-Arg-Asn-Leu-Asn-Asn-Phe-His-Arg-Phe-Ser-Gly-Met-Gly-Phe-
Gly-Pro-Glu-Thr-Pro-NH2; (pig)

-Val-Leu-Ser-Ala-Tyr-Trp-Lys-Asp-Leu-Asn-Asn-Tyr-His-Arg-Tyr-Ser-Gly-Met-Gly-Phe-
Gly-Pro-Glu-Thr-Pro-NH2; (sheep).

In formula (I) m preferably represents 1 and n preferably represents 0.

R¹-R⁴ preferably each represent hydrogen.

It will be appreciated that when (X-Y) represents -CH=CH- the compounds may exist as geometric isomers (cis or trans), and the invention encompasses all such isomers and mixtures thereof.

(X-Y) preferably represents -C≡C-.

Compounds of formula (I) may be prepared using the general condensation methods well known in the art of peptide chemistry. Thus in a further aspect the present invention provides a process for the preparation of compounds of formula (I) which comprises:

subjecting amino-acids, peptides or combinations thereof to condensation reaction(s) to form an amino-acid sequence in the order of formula (I) wherein active groups not participating in the condensation reaction are protected and at any appropriate stage of the reaction subjecting to cyclisation the structural units containing a peptide residue of formula (II) : 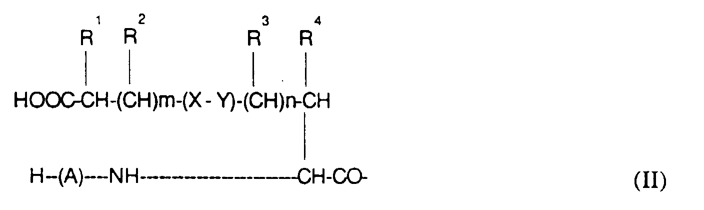 (wherein A, X-Y, m, n and R¹-R⁴ are as defined above)
and finally removing any protecting groups to give a compound of formula (I).

It will be understood that the protecting groups which may be used during the synthesis of compounds (I), including the intermediate fragments, can be chosen from the multitude of protecting groups known in peptide chemistry and the following examples of suitable groups are not intended to be exhaustive. In general, the protecting groups will be easily removable by hydrolysis, acid decomposition, reduction, aminolysis or hydrazinolysis, with the proviso that hydrogenolysis cannot be used for deprotection of peptides containing C-C unsaturated bonds.

Thus, amino protecting groups which may be employed include acyl such as formyl, acetyl, trifluoroacetyl, phthaloyl, benzenesulphonyl, p-toluenesulphonyl, o-nitrophenylsulphenyl or 2,4-dinitrophenylsulphenyl; aralkyl such as benzyl, diphenylmethyl or triphenylmethyl, each of which may be optionally substituted, for example, by C₁₋₄ alkoxy such a methoxy; aliphatic oxycarbonyl, such as t-butoxycarbonyl, diisopropylmethoxycarbonyl, trichloroethyloxycarbonyl or t-amyloxycarbonyl isobornyloxycarbonyl, adamantyloxycarbonyl; aralkyloxycarbonyl such as benzyloxycarbonyl (optionally substituted for example, by bromo, chloro, nitro, or methoxy), p-phenylazobenzyloxycarbonyl, p-(4-methoxyphenylazo)benzyloxycarbonyl, 2-phenylisopropoxycarbonyl, 2-tolylisopropoxycarbonyl, 2-(p-diphenyl)isopropoxycarbonyl or 9-fluorenylmethyloxycarbonyl; and enamine protecting groups formed by reacting the amino group with a 1,3-diketone such as benzoylacetone, acetylacetone or dimedone.

Particularly preferred amino protecting groups are t-butoxycarbonyl (BOC), benzyloxycarbonyl (Z) and especially 9-fluorenylmethyloxycarbonyl (FMOC).

The amino group or guanidino group in arginine may be protected by a nitro, tosyl, benzyloxycarbonyl, 4-methoxy-2,3,6-trimethylphenylsulphonyl or 2,2,5,7,8-pentamethylchroman-6-sulphonyl. Suitable protecting groups for the imino moiety in histidine include benzyl, trityl, benzyloxycarbonyl, tosyl, adamantyloxycarbonyl, 2,4-dinitrophenyl, benzyloxymethyl, 2,2,2-trifluoro-1-t-butyloxycarbonylaminoethyl or 2,2,2-trifluoro-1-benzyloxycarbonylaminoethyl.

A preferred imino protecting group is trityl. However the aforementioned guanidine and imino groups do not always require protection.

Carboxyl groups may be protected by conversion into an amide, hydrazide or ester. Esters may be derived, for example, from an alkanol, such as an optionally substituted C₁₋₄ alkanol, e.g. methanol, ethanol, t-butanol, cyanomethanol or 2,2,2-trichloroethanol; an aralkanol such a benzylalcohol, p-bromobenzylalcohol, p-chlorobenzylalcohol, p-methoxybenzylalcohol, p-nitrobenzylalcohol, 2,4,6-trimethylbenzylalcohol, benzhydrylalcohol, benzoylmethanol, p-bromobenzoyl methanol, p-chlorobenzoylmethanol or 9-fluorenyl methanol; side chain amide groups may be substituted for example by a 3,4-dimethoxybenzyl or bis-(p-methoxyphenyl) methyl group. A hydrazide may be substituted by a benzyloxycarbonyl, trichloroethyloxycarbonyl, trifluoroacetyl, t-butoxycarbonyl, trityl or 2-p-diphenylisopropoxycarbonyl group.

A hydroxyl group, for example, in serine, threonine or tyrosine may, if necessary, be protected by esterification or etherification. Esterifying groups which may be employed include C₁₋₄ alkanoyl such as acetyl; aroyl such as benzoyl; and aryloxycarbonyl or alkoxycarbonyl such as benzyloxycarbonyl or ethyloxycarbonyl. Suitable etherifying groups include benzyl, tetrahydropyranyl and t-butyl. Alternatively, a hydroxyl group may be protected by a 2,2,2-trifluoro-1-t-butyloxy-carbonylaminoethyl or a 2,2,2-trifluoro-1-benzyloxy-carbonylaminoethyl group. It will, however, be understood that protection of the hydroxyl groups is not always required.

As indicated above, compounds of formula (I) may be synthesised by carrying out, in an appropriate order, a series of condensation reactions between peptides and/or amino-acids.

At each stage of the reaction the amino and carboxyl groups of the peptides should be protected, deprotected, or activated as necessary to ensure that the correct peptide linkages are formed. Thus, for example, a peptide or amino-acid having a protected amino group and an activated carboxyl group may be reacted with a peptide or amino-acid having a free amino group and a protected carboxyl group. Such methods are described, for example by J. H. Jones, in "The Peptides" Vol. 1, (1979), pp. 66-100, E. Gross and J. Meienhofer Eds., Academic Press, New York, N.Y., USA).

The condensation reactions may be effected using either solid phase or liquid phase methods. In solid phase synthesis [see, for example G. Barany and R.B. Merrifield in "The Peptides", Vol. 2 (1979), pp. 1-284, E. Gross and J. Meienhofer Eds., Academic Press, New York, USA] the carbonyl group of a C-terminal amino acid in which the terminal amino group is protected is coupled to an insoluble carrier. The insoluble carrier may be selected from any material having a coupling ability to a reactive carbonyl group, for example, a halogenomethyl resin or the like, phenol resin, tert-alkyloxycarbonylhydrazide resin, benzhydrylamine resin, and the like can be used.

After removal of the amino-protecting group, further N-protected aminoacids are coupled sequentially through condensation reactions, in accordance with the amino acid sequence represented by general formula (I), to extend the chain of the polypeptide in a stepwise manner to the desired length following which the insoluble carrier is removed to yield the desired polypeptide.

Liquid-phase synthesis may be carried out for example using methods described by M. Mutter and E. Bayer in "The Peptides", Vol. 2 (1979), pp. 285-332, E. Gross & J. Meienhofer Eds, Academic Press, New York, N.Y., USA.

The following activation and condensation methods may generally be employed in either solid or liquid phase synthesis.

A carboxyl group may be activated in conventional manner, for example, as an acid anhydride (preferably a mixed anhydride in liquid phase synthesis or a symmetrical anhydride for solid phase synthesis), acid halide, acid azide or an activated ester such as a cyanomethyl ester, thiophenyl ester, p-nitrophenyl ester, p-nitrothiophenyl ester, 2,4,6-trichlorophenyl ester, pentachlorophenyl ester, pentafluorophenyl ester, N-hydroxyphthalimido ester, 8-hydroxypiperidine ester, N-hydroxypiperidine ester, N-hydroxysuccinimide ester, N-hydroxybenzotriazole ester, or a haloformate, e.g. a chloroformate; or the carboxyl group may be activated using a carbodiimide, N,N ¹-carbonyldiimidazole, Woodward-K reagent, Castro's reagent or as an isoxazolium salt.

Condensation of an amino group with a reactive carboxyl group may be carried out in the presence of a basic compound, in a suitable solvent. Basic compounds which may be employed include organic bases such as triethylamine, trimethylamine. N,N-diisopropylethylamine, pyridine, N,N-dimethylaniline, N-methylmorpholine, 1,5-diazabicyclo(4,3,0)-5-nonene (DBN), 1,5-diazabicyclo(5,4,0)-5-undecene (DBU), 1,4-diazabicyclo(2,2,2)-octane (DABCO) and the like; and inorganic bases such as potassium carbonate, sodium carbonate, potassium hydrogen carbonate, sodium hydrogen carbonate and the like. Solvents which may be employed include any which are known in the peptide art as being suitable for this type of condensation reaction, for example anhydrous or aqueous DMF, dimethyl sulfoxide (DMSO), pyridine, chloroform, dioxane, dichloromethane, THF, ethyl acetate, N-methylpyrrolidone and hexamethylphosphoric triamide (HMPA) and the like and mixtures thereof. The reaction temperature may be within usual temperature range being employed in this type of condensation reaction, and generally it is selected suitably from the range of about - 40°C to about 60°, preferably, from about -20°C to about 40°. The reaction may generally be carried out within about several minutes to 120 hours.

When the carboxyl group is activated as a mixed acid anhydride this is generally formed in situ with an alkyl halocarboxylic acid for example methyl chloroformate, methyl bromoformate, ethyl chloroformate, ethyl bromoformate, isobutyl chloroformate and the like. The condensation is effected in a suitable solvent, in the presence of a base, for example, an organic base such as triethylamine, trimethylamine,N,N-diisopropylethylamine, pyridine, N,N-dimethylaniline, N-methylmorpholine, DBN, DBU or DABCO and the like, or an inorganic base such as potassium carbonate, sodium carbonate, potassium hydrogen carbonate, or sodium hydrogen carbonate. Solvents which may be employed include halogenated hydrocarbons such as methylene chloride, chloroform, dichloroethane and the like, aromatic hydrocarbons such as benzene, toluene, xylene and the like, ethers such as diethyl ether, THF, dimethoxyethane and the like, esters such as methyl acetate, ethyl acetate and the like, and aprotic polar solvents such as DMF, DMSO, HMPA and the like. The reaction is carried out, generally under conditions of about -20° to 100°C, preferably at about -20° to 50°C.

When condensation is carried out in the presence of a suitable condensing agent, for example a carbodiimide, N,N-carbonyldiimidazole, Woodward-K reagent, Castro's reagent or the like, the condensing agent is preferably employed in an amount from equimolar to about 5 times the molar quantity of the starting material and the reaction is performed in a suitable solvent for example a halogenated hydrocarbon such as dichloromethane, chloroform, carbon tetrachloride, tetrachloroethane or the like; an ether such as dioxane, THF, dimethoxyethane or the like, a ketone such as acetone, methyl ethyl ketone or the like; acetonitrile, ethyl acetate, DMF, dimethylacetamide, DMSO or the like. Preferably the condensation is carried out in an anhydrous solvent, and at reaction temperature of about, generally -10° to 60°C, preferably about 0°C to room temperature.

Cyclisation of the peptide residue (II) 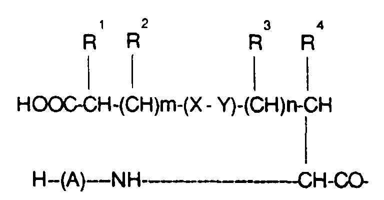 may be effected by activation of the carboxyl group followed by condensation with the terminal amino group of fragment (A) using one of the condensation methods previously described. In this reaction the carboxyl group is preferably activated using benzotriazol-1-yloxy-tris (dimethylamino) phosphonium hexafluorophosphate (Castro's reagent, BOP) using DMF as a solvent at -5° -0°C in the presence of N-methyl morpholine. During this reaction the hydroxyl groups of Ser and Thr should preferably be protected.

The peptide residue (II) may itself be formed by condensation of a peptide residue

H-(A)-OH

with a compound of formula (III) 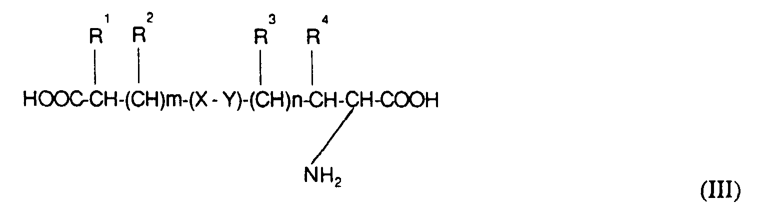 (wherein R¹-R⁴, X, Y, m and n are as defined for formula (I)) optionally followed by condensation with further amino-acids or peptides.

The condensation may be carried out using the methods well known in peptide synthesis, for example, as described above. It will be appreciated that non-participating active groups in the peptide residue and the compound (III) should be protected as previously described.

Following condensation of the peptide residue H-(A)-OH with a compound of formula (III), the resulting compound of formula (II) may be subjected to condensation with further amino-acids or peptides to introduce the amino-acid residues from 8 upwards. Preferably however, the compound (II) is cyclised as described above, prior to the addition of further peptide residues.

In a preferred embodiment, the peptide residue H-(A)-OH represents H-Ser-Asn-Leu-Ser-Thr-OH and the compound of formula (III) represents 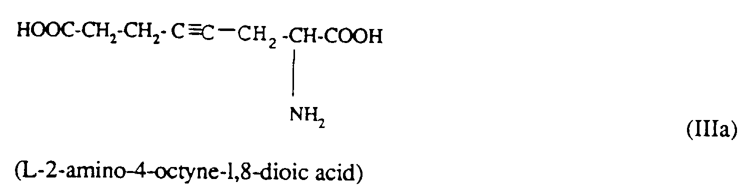 or 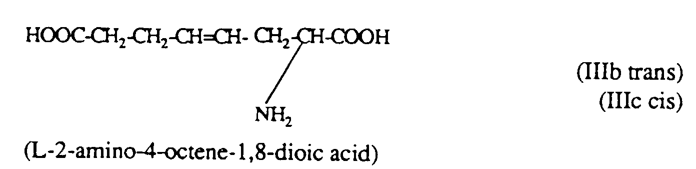 wherein non-participating active groups are protected.

L-2-Amino-4-octyne-1,8-dioic acid (IIIa) and the corresponding protected derivative (S)-2-(9-fluorenylmethyloxycarbonylamino)-4-octyne-1,8-dioic acid 1-methyl ester are novel compounds and as such form a further aspect of this invention.

L-2-amino-4-octyne-1,8-dioic acid (III a) may be prepared according to reaction scheme 1, trans-L-amino-4-octene- 1,8-dioic acid (IIIb) may be prepared according to reaction scheme 2, or by other methods such as reduction of the acetylenic analogue (IIIa) with sodium in liquid ammonia; cis-L-2-amino-4-octene-1,8-dioic acid (IIIc) can be prepared by partial hydrogenation of (IIIa) in the presence of a poisoned catalyst, for example palladium on barium sulphate/quinoline or by other methods reported in the literature, for example as described in J. Org. Chem., 54, 4224 (1989).

Other compounds of formula (III) may be prepared by analogous methods, entailing condensation of compound (IV) 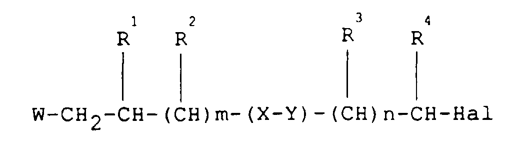 where W is a protected carboxy group or a group which can be transformed into carboxy group, for example a protected CH₂OH group, and Hal is an halogen atom, while R¹-R⁴ and X-Y have the some meaning as in compounds of formula (I), with a reagent useful for the synthesis of optically active aminoacids such as chiral 2,5-dihydro-3,6-dimethoxy-2-isopropylpyrazine (Schollkopffs reagent), chiral 1-benzoyl-2-t-butyl-3-methyl-4-imidazolidinone and the like.

It will be appreciated that compounds (III) contain one or more chiral centres. The present invention covers all optical isomers of these compounds in their fully and partially resolved forms and in the form of racemic mixtures.

Protecting groups introduced at various stages of the synthesis may be removed at any appropriate point, either at an intermediate stage or as the final step. In general removal of the protecting groups may be effected by standard methods such as acid or base hydrolysis, acid decomposition, reduction, aminolysis, hydrazinolysis or hydrogenolysis, with the proviso that hydrogenolysis should not be used for deprotection of peptides containing an unsaturated carbon-carbon bond. Thus final-stage deprotection of a compound of formula (I) is preferably effected by hydrolysis, most preferably under acidic conditions.

Deprotection under acidic conditions is generally effected using a strong acid such as trifluoroacetic acid, hydrochloric acid, hydrogen fluoride, methanesulfonic acid, hydrobromic acid or the like, optionally in the presence of a co-reagent such as trimethylsilyl bromide and of the so called "carbocation scavengers" such as thioanisole, methyl ethyl sulphide, m-chresol, phenol and the like. In general a large excess of the acid may be employed. In order to selectively remove amino-protecting groups, trifluoroacetic acid or hydrochloric acid are preferably employed.

The thus obtained deprotected polypeptides can be separated and purified by methods usually employed in separation of peptides, for example solvent extraction, distribution, chromatography and the like. Preferably separation and purification are performed using High Performance Liquid Chromatography (HPLC), for example using a reversed-phase silica gel stationary phase and mixtures of water/acetonitrile/trifluoroacetic acid as the mobile phase.

Compounds of formula (I) have been found to exhibit hypocalcaemic activity in *vivo* (rat) and they are expected to have utility in medicine, for example in the treatment of conditions such as Paget's disease (osteitis deformans), osteoporosis, including postmenopausal osteoporosis, Sudeck's disease and various hypercalcaemic conditions (see for example the Physician's Desk Reference, 42nd Edition, 1988, pages 1796 and 1797).

In a further aspect, therefore, the present invention provides compounds of formula (I) for use in therapy, for example, in the treatment of osteoporosis, Paget's disease, Sudeck's disease and hypercalcaemic conditions.

In therapeutic use the compounds of the present invention are usually administered in a standard pharmaceutical composition.

The present invention therefore also provides pharmaceutical compositions comprising a compound of formula (I) or a pharmaceutically acceptable salt thereof, and a pharmaceutically acceptable carrier.

Compounds of formula (I) may be formulated for administration by any convenient route, for example, parenteral, nasal, buccal, sublingual, transdermal, rectal or vaginal. Whilst oral administration is not generally appropriate for calcitonin derivatives, some oral dosage forms may be suitable, for example, enterically coated oral compositions. Preferably the compounds of the invention are formulated for transmucosal delivery, for example, by administration to nasal, sublingual, buccal, rectal, vaginal or colonic mucosa or transdermal delivery. Such compositions can take the form of drops, aerosols, tablets, capsules, wafers, powders, gels, ointments, inserts, suppositories, pessaries, patches, membranes, and enterically coated solid oral compositions.

When the composition is intended for delivery to the nasal mucosa, particular dosage forms are solutions, aerosols, drops, gels and powders. Particular dosage forms for buccal and sublingual administration are gels, suspensions, tablets, patches, powders, ointments, solutions, aerosols and wafers.

Aerosol formulations typically comprise a solution or fine suspension of the active substance in a physiologically acceptable aqueous or non-aqueous solvent and are usually presented in single or multidose quantities in sterile form in a sealed container. The sealed container can take the form of a cartridge or refill for use with an atomising device, or it can take the form of a unitary dispensing device such as a single dose nasal inhaler or an aerosol dispenser fitted with a metering valve and which is intended for disposal once the contents of the container have been exhausted. Where the dosage form comprises an aerosol dispenser, it will contain a propellant which can be a compressed gas such as compressed air or an organic propellant such as a fluorochlorohydrocarbon. The aerosol dosage forms can also take the form of a pump-atomiser. The atomising or dispensing devices for dispensing aerosol sprays typically are designed to dispense particles of a size greater than 10 micrometres. In order to ensure that significant quantities of the composition remain in contact with the oral or nasal mucosa, and are not inhaled, the particles suitably are approximately 10-160 micrometres in size.

When the composition is intended to be administered as a liquid spray, the viscosity of the liquid composition will be adjusted as necessary according to known methods to ensure that the composition is sprayable.

Particular dosage forms for vaginal and rectal administration include pessaries, suppositories, solutions, foams, suspensions, gels, ointments, tablets and soft gelatin capsules.

Compositions for rectal or vaginal administration are generally presented as a solid suppository or a semisolid or liquid formulation filled into a soft gelatin capsule. It will be appreciated therefore that the excipients for use in such suppository or capsule formulations will be selected and if necessary admixed to give a formulation of the desired consistency at room and body temperatures. Thus, the suppository base or carrier may for example comprise one or more components selected from an oil, a fat, a polyglycolysed glyceride and a polyethylene glycol. The oil and/or fat preferably comprises one or more triglycerides as the main component, such as coconut oil, fractionated coconut oil (e.g. Miglyol) palm kernel oil, palm fat, cocoa butter or lard. Examples of hard fat suppository bases include Witepsol and Suppocire. A saturated or unsaturated polyglycolysed glyceride may be for example a saturated polyglycolysed glyceride consisting of C₈₋₁₈ glycerides and polyethylene glycol esters such as are available under the trade name Gelucire e.g. Gelucire 35/10, 37/02 or 44/14; a saturated polyglycolysed C₈-C₁₀ glyceride such as that available under the trade name Labrasol; or an unsaturated polyglycolysed glyceride consisting of C₁₆-C₂₀ glycerides and polyethylene glycol esters such as those available under the trade name Labrafils e.g. Labrafil WL 2609 BS or M 2125 CS. For use in a capsule formulation the polyethylene glycol component is preferably liquid at room temperature such as polyethylene glycol-200, 300, 400 or 600, whereas for a solid suppository a polyethylene glycol of higher molecular weight is preferred. The relative proportions of the excipients will of course depend inter alia on the consistency of the formulation required.

Compositions containing a polyglycolysed glyceride optionally with a polethylene glycol can also be adapted for oral administration e.g. in hard or soft gelatin capsules, which are preferably enterically coated.

When the composition is enterically coated and is intended for oral administration, typically it can take the form of a tablet or capsule coated with a coating agent which ensures passage of the calcitonin through the stomach and its subsequent release preferably in the colon. Suitable coating agents include anionic polymers such as acrylic acid/methacrylic acid ester copolymers (e.g. Eudragit S).

Typical parental compositions consist of a solution or suspension of the compound or pharmaceutically acceptable salt in a sterile aqueous carrier or parentally acceptable oil, for example polythylene glycol, polyvinyl pyrrolidone, lecithin, arachis oil or sesame oil. Alternatively, the solution can be lyophilised and then reconstituted with a suitable solvent just prior to administration.

A composition in the form of a capsule can be prepared using routine encapsulation procedures. For example, pellets containing the active ingredient can be prepared using standard carriers and then filled into a hard gelatin capsule; alternatively, a dispersion or suspension can be prepared using any suitable pharmaceutical carrier(s), for example aqueous gums, celluloses, silicates or oils and the dispersion or suspension then filled into a soft gelatin capsule.

A composition in the form of a tablet can be prepared using any suitable pharmaceutical carrier(s) routinely used for preparing solid formulations. Examples of such carriers include magnesium stearate, starch, lactose, sucrose and cellulose.

The solvents or liquid carriers used in the present formulations are preferably aqueous but can also be chosen from the physiologically acceptable non-aqueous solvents. Examples of non-aqueous solvents or carriers are alcohols, particularly polyhydroxy alcohols such as propylene glycol and glycerol, and vegetable and mineral oils. Such non-aqueous solvents or carriers can be added in various concentrations to water to form solutions, oil-in-water emulsions and water-in-oil emulsions. The solvent preferably is water.

In addition to a solvent or carrier, the liquid formulations of the present invention can contain excipients such as antioxidants, stabilisers, preservatives, agents for adjusting viscosity (e.g. Carbapol, Keltrol or cellulose derivatives),agents for adjusting tonicity, (e.g. sodium chloride, glycine or mannitol) and buffering agents.

When a preservative is employed, it is chosen such that in the quantities needed to preserve the formulation, it does not cause irritation of the nasal mucosa. This is particularly important when the formulation is intended to be administered on a long term basis, for example when used in the treatment of post-menopausal osteoporosis. Suitable preservatives are the alkyl p-hydroxybenzoates (parabens) such as methyl p-hydroxybenzoate and propyl p-hydroxybenzoate. Alternatively benzyl alcohol may be employed.

The pH of the liquid compositions of the present invention can vary within a broad range according to the chemicophysical properties of the different ingredients in the compositions. However, suitably the pH of the composition is in the range from pH 3 to 8, preferably from approximately pH 4 to approximately pH 7. In order to regulate the pH and maintain a suitable value, a buffering agent may be included in the composition. Examples of buffering agents which may be used include citrates, for example a mixture of citric acid and sodium citrate, acetates and phosphates. In addition to a buffering agent such as those described hereinabove, an alkali metal hydroxide e.g. sodium hydroxide may be incorporated to regulate the pH.

Compositions according to the invention, in particular those for administration via the transmucosal route, may also contain an agent to enhance absorption. Such agents are well known in the calcitonin art and include choline esters, acyl carnitines, aldoses, glycosamines, ascorbates, salicylates, alpha-cyclodextrins, pyroglutamate esters, chelating agents, ethanol, benzyl alcohol, polyethylene glycol 400 and surfactants. Preferably the absorption promoter is one which does not cause irritation at the site of administration, for example glycyrrhizinic acid or a salt thereof e.g. sodium or potassium glycyrrhizinate or ammonium glycyrrhizinate.

The compositions can also contain a protease inhibitor, preferably a non-surfactant protease inhibitor, for example as described in EP 127535.

In general, the above-mentioned compositions can be made according to well known pharmaceutical procedures, see for example Remington's Pharmaceutical Sciences, 17th Edition, Mack Publishing Company, 1985. Soft gelatin capsules may be prepared for example as described in WO 84/03417 or EPA 122463. Wafer formulations may be prepared for example as described in PCT/GB91/00651. Thus for example the active ingredient may be incorporated into the wafer mix prior to forming the wafer, or applied to the wafer in the form of a layer or a spray.

The compositions will be administered to the patient in dosages which contain an amount of calcitonin effective to treat the disease in question.

The quantity of pharmacologically active substance in a unit dose of the compositions of the present invention will vary according to the potency of the calcitonin and the nature of the composition. However, in general, a unit dose of a composition intended for human use typically contains between 5 and 200 International Units (I.U.) of a compound of the invention. A typical dosage regimen is from 5 to 200 I.U. per day which may be administered in a single dose or in divided doses.

The term "International Unit" refers to the appropriate International Reference Preparation (I.R.P.) of human, salmon or porcine calcitonins, or elcatonin, established by the National Institute for Biological Standards and Control, Blanche Lane, South Mimms, Potters Bar, Hertfordshire, EN6 3QG, United Kingdom.

When the formulation is a liquid formulation, particularly a spray, the volume of a unit dose typically is in the range 50 to 130 mcl.

The invention is further illustrated by the following non-limiting examples:

### Preparation of Intermediates

### Preparation 1

### 2-(4-Pentynoxy)tetrahydropyran

To a solution of 56 g of 4-pentynol in 1.5 1 of anhydrous diethyl ether, 223.8 g of 3,4-dihydro-2H-pyran and 1 g of p-toluenesulfonic acid monohydrate were added under magnetic stirring. After 4 hours at room temperature a 1.7 N solution of ammonia in methanol was added until pH 9 was reached. The obtained white precipitate was filtered off and the filtrate was evaporated to dryness. The oily residue was distilled under vacuum to afford 87 g (88%) of the title compound as a colourless oil, b.p. 55-56°C (0.4 mm Hg).

NMR (CDCl₃): δ_{H} = 4.57 (m, 1H, CHO); 4.0-3.30 (m, 4H, 2 CH₂O); 2.50-2.15 (m, 2H, C-CH₂); 2.10-1.40 (m, 9H, **H**C≡C-CH₂-C **H**₂- and -C**H**₂-C**H**₂-C**H**₂-CH)
MS (E.I., 70 eV, 1.5 mA) m/z = 168 (M⁺), 85 (C₅H₉O)⁺, 101 (C₅H₉O₂)⁺.

### Preparation 2

### 6-(2-Tetrahydropyranyloxy)-2-hexynol

A solution of 70.2 g of 2-(4-pentinoxy)tetrahydropyran in 420 ml of anhydrous tetrahydrofuran was chilled to -70°C under nitrogen and 264 ml of a 1.6 N solution of butyl lithium in hexane were dropped under magnetic stirring, maintaining the internal temperature between -70°C and -60°C. After heating to 10°C, 27.7 g of powdered paraformaldehyde were added and stirring was continued for 40 min at room temperature. The mixture was then refluxed for 1.5 hours. After cooling, 1200 ml of water were added, the organic solvents were removed in vacuo and the remaining aqueous mixture was extracted twice with ethyl acetate (1500 ml each). The two organic phases were collected, dried over anhydrous sodium sulfate and evaporated to dryness. Distillation of the residue under vacuum afforded 65.4 g (77%) of the title compound as a colourless oil, b.p. 102-104°C (0.3 mmHg).

NMR (CDCl₃): δ_{H} = 4.60 (m, 1H, CHO); 4.25 (m, 2H, C**H**₂OH) 4.05-3.30 (m, 4H, 2 CH₂O); 2.50-2.15 (m, 2H, C≡C-C**H**₂); 2.05-1.35 (m, 9H, C≡C-CH₂-C**H**₂, OH, -C**H**₂-C**H**₂-C**H**₂)
MS (E.I., 70 eV, 1.5 mA) m/z = 197 (M-H)⁺, 167 (M-CH₃O)⁺, 101 (C₅H₉O₂)⁺, 85 (C₅H₉O)⁺.

### Preparation 3

### 2-(6-Chloro-4-hexynoxy)tetrahydropyran

To an ice cold solution of 64 g of 6-(2-tetrahydropyranyloxy)-2-hexynol in 1000 ml of anhydrous methylene chloride, 220 ml of N-ethyldiisopropylamine were added under stirring. 50.2 ml of mesyl chloride were then dropped at 0°C and stirring was continued for 15 min. After addition of 27.5 g of lithium chloride, the resulting suspension was stirred overnight at room temperature. The insoluble material was filtered off, 1200 ml of water were added and the organic solvent was evaporated under vacuum. After extraction of the remaining aqueous mixture with 1200 ml of diethyl ether, the organic phase was washed twice with a 3% solution of NaCl (1200 ml each), dried over anhydrous sodium sulfate and evaporated under vacuum. The residue was chromatographed over silica gel (ligroindiisopropyl ether 6:4) to afford 57.9 g (82.7%) of the title compound as an oil, b.p. 86°C (4 mm Hg).

NMR (CDCl₃): δ_{H} = 4.58 (m, 1H, CHO); 4.12 (t, J=2Hz, 2H, -CH₂Cl); 4.05-3.30 (m, 4H, 2 CH₂O); 2.50-2.15 (m, 2H, C≡C-CH₂); 2.00-1.30 (m, 8H, C≡C-C**H**₂-C**H**₂, -C**H**₂-C**H**₂-C**H**₂)
MS (E.I., 70 eV, 1.5 mA) m/z = 215 (M-H)⁺, 180 (M-HCl)⁺, 167 (M-CH₂Cl)⁺, 85 (C₅H₉O)⁺.

### Preparation 4

### [S-(R*,S*)]-2-Isopropyl-2,5-dihydro-3,6-dimethoxy-5-[6-(2--tetrahydropyranyloxy)-2-hexynyl]pyrazine

To a solution of 7.14 ml of (R)-(-)-2,5-dihydro-3,6-dimethoxy-2-isopropylpyrazine in 240 ml of anhydrous tetrahydrofuran, 9.77 ml of 1,3-dimethyl-3,4,5,6-tetrahydro-2-(1H)-pyrimidinone were added. The solution was stirred and chilled to -70°C under a nitrogen atmosphere and 27.5 ml of a 1.6 N solution of butyl lithium in hexane were dropped in, mantaining the temperature at -70°C. After 15 min a solution of 26.0 g of 2-(6-chloro-4-hexynoxy)tetrahydropyran in 30 ml of anhydrous tetrahydrofuran was added dropwise at -70°C and the reaction was stirred for 3.5 hours at the same temperature. After evaporation of the solvent, the crude oily residue was diluted with 480 ml of diethyl ether and with 480 ml of a 3% solution of NaCl. The organic phase was separated, washed with 480 ml of 3% NaCl and twice with water (480 ml each). Treatment with anhydrous sodium sulfate and evaporation to dryness, gave 31.5 g of an oil which was chromatographed over silica gel (step gradient of hexane-diethyl ether 90:10, 85:15, 80:20) affording 5.6 g (38.4%) of the title compound as an oil. Rf=0.3 (silica gel plate, eluent: petroleum ether-diethyl ether 8:2) [α]_{D}=+ 44.8 (c=1, dichloromethane).

NMR (CDCl₃): δ_{H} = 4.60 (m, 1H, CHO); 4.20-3.30 (m, 6H, 2 CH₂O, =N-C**H**); 3.70 (s, 6H, -OCH₃); 2.65 (m, 2H, CHC**H**₂-C≡C); 2.45-2.05 (m, 3H, CH₃-C**H** and C≡C-C**H**₂); 1.95-1.35 (m, 8H, C≡C-CH₂ C**H**₂ and -C**H**₂-C**H**₂-C**H**₂-); 1.05 and 0.68 (d, J=7Hz, 6H, (C**H**₃)₂CH).
MS (E.I., 70 eV, 1.5 mA) m/z = 364 (M⁺), 321 (M-C₃H₇)⁺, 278 (M-C₅H₁₀O)⁺, 182 (M-C₉H₁₄N₂O₂)⁺.

### Preparation 5

### (S)-2-(9-Fluorenylmethyloxycarbonylamino)-4-octyne-1,8-dioic acid 1-methyl ester

[S-(R*,S*)]-2-Isopropyl-2,5-dihydro-3,6-dimethoxy-5-[6-(2-tetrahydropyranyloxy)-2-hexynyl]pyrazine (3.86 g) was suspended in 77 ml of 0.25 N HCl and stirred at 0°C for 5 hours. After evaporation of the solvent, the resulting oil was dissolved in anhydrous toluene and evaporated to dryness. This operation was repeated three times. The crude product was dissolved in 210 ml of anhydrous methylene chloride, and treated with 2.96 ml of triethylamine and with 17.12 g of 9-fluorenylmethyl succinimidyl carbonate. After 4.5 hours, further 2.14 g of the latter reagent and 0.75 ml of triethylamine were added and stirring was continued for 6 hours. After addition of 370 ml of 0.1 N HCl the phases were separated and the aqueous layer was extracted again with 105 ml of methylene chloride. The two organic phases were collected and washed with 150 ml of water, dried over anhydrous sodium sulfate and evaporated to dryness. The oily residue was dissolved in 800 ml of acetone, cooled to 0°C, and treated dropwise under vigorous stirring with a solution of chromic anhydride in sulfuric acid (15 ml) (Jones reagent: 4 g of CrO₃ were dissolved in 3.5 ml of concentrated sulfuric acid. Water was then added to 15 ml total volume). The excess of unreacted CrO₃ was reduced with isopropanol and the insoluble material was filtered off on a Celite pad. The filtrate was concentrated to 225 ml and 340 ml of water were added. The solution was extracted three times with diethyl ether (180 ml each). The organic phases were collected and extracted five times with 7% aqueous NaHCO₃ (180 ml each). The collected aqueous phases were acidified to pH=1 with 10% HCI, and extracted with 3x150 ml of methylene chloride. The collected organic phases were evaporated to dryness and the residue was dissolved in 25 ml of ethyl acetate at 50°C. Upon addition of 150 ml of hexane a white solid precipitated on cooling, affording 2.42 g (54%) of the title compound as a white solid. m.p. 148-152°C [alfa]_{D}=+17.9° (c=1, tetrahydrofuran).

NMR (CDCl₃): δ_{H} = 7.88-7.25 (m, 8H, Ar-H); 6.05 (bs, 1H, COOH); 5.72 (d, J=8.5, N**H**); 4.80-4.15 (m,.4H, >C**H**-C**H**₂-OCONHC**H**); 3.78 (s, 3H, COOCH₃); 3.00-2.25 (m, 6H, C**H**₂-C≡C-C**H**₂-C**H**₂-).
MS (E.I., 70 eV, 15 mA) m/z = 362 (M-C₂H₃O₂)⁺, 178 (M-C₁₀H₁₃NO₆)⁺.

### Preparation 6

### Fmoc-Thr(t-Bu)-OBzl

To a suspension of 24 g of Fmoc-Thr(t-Bu)-OH in 50 ml of 7% aqueous sodium bicarbonate, a solution of 9.7 ml of benzyl bromide and 15.8 g of hexadeciltributylphosphonium bromide in 50 ml of methylene chloride was added under vigorous mechanical stirring. After 2 hours, 9 g of sodium bicarbonate, 50 ml of methylene chloride and 50 ml of water were added. The reaction mixture was then stirred at room temperature overnight. After separation of the two phases, the aqueous layer was extracted with 100 ml of methylene chloride. The organic phases were then collected, washed with 2N hydrochloric acid (2x200 ml), dried over anhydrous sodium sulfate and evaporated to dryness. The residue was flash-chromatographed over silica gel (cyclohexane-ethyl acetate 8:2). The appropriate fractions were collected and the solvent was evaporated under vacuum. The residue was recrystallized from n-hexane, affording the title compound as a white solid (28.1 g, 95% yield). Rf = 0.5 (silica gel plate, eluent cyclohexane-ethyl acetate 8:2).
MS(FAB, 8-10 kV, 2mA) m/z=488(MH⁺), 432(MH-C₄H₈)⁺

### Preparation 7

### Fmoc-Ser(t-Bu)-Thr(t-Bu)-OBzl

A) A solution of 27.3 g of Fmoc-Thr(t-Bu)-OBzl in 580 ml of a 10% solution of diethylamine in anhydrous dimethylformamide was stirred at room temperature for 30 min. After evaporation of the solvent under vacuum, the residue was dissolved in 11 of diethyl ether and then extracted three times with 1% HCI (600 ml each). The aqueous phases were collected, adjusted to pH=8 with sodium bicarbonate and extracted three times with methylene chloride (600 ml each). The organic phases were collected, dried over anhydrous sodium sulfate and evaporated to dryness. The residue [H-Thr(t-Bu)-OBzl] was dissolved in anhydrous tetrahydrofuran and directly used in the following reaction.
B) To a cooled solution (-25°C) of 23.6 g of Fmoc-Ser(t-Bu)-OH in 210 ml of anhydrous tetrahydrofuran, 6.8 ml of N-methylmorpholine and 8.0 ml of isobutyl chloroformate were successively added. The reaction was then stirred for 3 min keeping the temperature between -15°C and -12°C. The precooled solution A (-25°C) was poured into the reaction mixture and stirring was continued for 45 min at the same temperature and for 1 hour at 0°C. After filtration of the insoluble material, the filtrate was evaporated to dryness. The residue was dissolved in 1.5 1 of ethyl acetate and washed successively with 3x1 l 10% citric acid, 1 l brine, 5x1 l 5% aqueous sodium bicarbonate and 1 l brine. The organic phase was dried over anhydrous sodium sulfate, evaporated to dryness and the residue was flash-chromatographed over silica gel (step gradient with cyclohexane-ethyl acetate 6:4, ethyl acetate) affording the title compound as a white sticky solid (35.2 g, 99%) Rf = 0.25 (silica gel plate, eluent cyclohexane-ethyl acetate 8:2).
   MS(FAB, 8-10kV, 2mA) m/z=631(MH⁺), 575(MH-C₄H₈)⁺, 519(MH-2C₄H₈)⁺.

### Preparation 8

### Fmoc-Leu-Ser(t-Bu)-Thr(t-Bu)-OBzl

A solution of 30.0 g of Fmoc-Ser(t-Bu)-Thr(t-Bu)-OBzl in 510 ml of a 10% solution of diethylamine in anhydrous dimethylformamide was stirred for 30 min at room temperature. After evaporation to dryness, the residue was dissolved in 250 ml of anhydrous dimethylformamide and 6.43 g of N-hydroxybenzotriazole were added under magnetic stirring at room temperature. 24.71 g of N-fluorenylmethyloxycarbonyl-L-leucine pentafluorophenyl ester (Fmoc-Leu-OPfp) were then added and stirring was continued for 1 hour.

After evaporation of the solvent, the residue was triturated with 580 ml of ethyl acetate and the solid material was filtered off. The filtrate was washed with 3x350 ml of 10% citric acid, 350 ml of brine, 6x350 ml of 5% sodium bicarbonate, 350 ml of brine. The organic phase was dried over anhydrous sodium sulfate, evaporated to dryness and flash-chromatographed over silica gel (step gradient with cyclohexane-ethyl acetate 80:20, 70:30, 60:40). The appropriate fractions were collected and evaporated to dryness giving 35.7 g (100% yield) of the title compound as a white sticky solid, Rf = 0.49 (silica gel plates, eluent cyclohexane-ethyl acetate 6:4).
MS(FAB, 8-10kV, 2mA) m/z=744(MH⁺), 688(MH-C₄H₈)⁺, 632(MH-2C₄H₈)⁺

### Preparation 9

### Fmoc-Asn-Leu-Ser(t-Bu)-Thr(t-Bu)-OBzl

A solution of 24.9 g of Fmoc-Leu-Ser(t-Bu)-Thr(t-Bu)-OBzl in 345 ml of a 10% solution of diethylamine in anhydrous dimethylformamide was stirred at room temperature for 30 min. After evaporation of the solvent to dryness, the residue was dissolved in 186 ml of anhydrous dimethylformamide and 4.54 g of N-hydroxybenzotriazole were added under magnetic stirring at room temperature. 23,1 g of N-Fmoc-L-asparagine pentafluorophenyl ester were then added, and stirring was continued for 2.5 hours. After evaporation to dryness, the residue was dissolved in 1l of methylene chloride and the solid material was filtered off. The filtrate was washed successively with 3x500 ml of 10% citric acid-brine 85:15, 500 ml of brine, 5x500 ml of sodium bicarbonate and 500 ml of brine.

After anhydrification and evaporation to dryness, the residue was flash-chromatographed over silica gel (step gradient with methylene chloride and methylene chloride-methanol 98:2, 95:5). After evaporation of the collected fractions the residue was triturated with 400 ml of hexane, affording the title compound as a white solid (23.3 g, 80%). Rf = 0.52 (silica gel plates, ethyl acetate-cyclohexane-acetic acid-water 10:10:2:1).
MS(FAB, 8-10kV, 2mA) m/z=858(MH⁺), 802(MH-C₄H₈)⁺, 746(MH2C₄H₈)⁺

### Preparation 10

### H-Asn-Leu-Ser(t-Bu)-Thr(t-Bu)-OBzl

A solution of 24.28 g of Fmoc-Asn-Leu-Ser(t-Bu)-Thr(t-Bu)-OBzl in 295 ml of a 10% solution of diethylamine in anhydrous dimethylformamide was stirred at room temperature for 30 min and then evaporated to dryness. The residue was flash-chromatographed over silica gel (step gradient with ethyl acetate-methanol 95:5, 90:10, 80:20) affording the title compound (27.5 g, 97%) as a white sticky solid. Rf = 0.31 (silica gel plates, dichloromethane-methanol 9:1).
MS(FAB, 8-10kV, 2mA) m/z=636(MH⁺), 580(MH-C₄H₈)⁺, 524(MH-2C₄H₈)⁺

### Preparation 11

### Fmoc-Ser(t-Bu)-Asn-Leu-Ser(t-Bu)-Thr(t-Bu)-OBzl

A solution of 9.58 g of Fmoc-Ser(t-Bu)-OH and 4.60 g of pentafluorophenol in 230 ml of ethyl acetate was stirred at 0°C and 5.16 g of dicyclohexylcarbodiimide were added. After 1 hour at 0°C and 1 hour at room temperature, the solid material was filtered off and the filtrate was evaporated to dryness. The residue was triturated twice with diethyl ether and the precipitate was filtered off. The filtrate was evaporated to dryness, affording a white solid (14.5 g) which was dissolved in 50 ml of anhydrous dimethylformamide and directly used in the next operation (Solution A).

12.5 g of H-Asn-Leu-Ser(t-Bu)-Thr(t-Bu)-OBzl and 2.66 g of N-hydroxybenzotriazole were dissolved in 100 ml of anhydrous dimethylformamide and Solution A was then added under magnetic stirring. After 4 hours at room temperature, the reaction mixture was evaporated to dryness, the residue was dissolved in 500 ml of methylene chloride and washed successively with 3x250 ml of citric acid - brine 85:15, 250 ml of brine, 5x250 ml of 5% sodium bicarbonate and 250 ml of brine. The organic phase was dried over anhydrous sodium sulfate, evaporated to dryness and the residue was flash-chromatographed over silica gel (step gradient with methylene chloride and methylene chloride-methanol 98:2). Evaporation of the appropriate collected fractions afforded the title compound (19.63 g, 99%) as a white solid, Rf = 0.28 (silica gel plates, eluent dichloromethane-methanol 95:5).
MS(FAB, 8-10kV, 2mA) m/z=1002(MH)⁺, 946(MH-C₄H₈)⁺, 889(MH-2C₄H₈)⁺

### Preparation 12

### Fmoc-Ser(t-Bu)-Asn-Leu-Ser(t-Bu)-Thr(t-Bu)-OH

Into a solution of 11.8 g of Fmoc-Ser(t-Bu)-Asn-LeuSer(t-Bu)-Thr(t-Bu)-OBzl in 137 ml of dimethylformamide and 550 ml of methanol, containing 4.7 g 10% palladium on charcoal, hydrogen was bubbled for 15 min at room temperature; 2.7 ml of 50% aqueous acetic acid were then added and bubbling continued for further 15 min. The catalyst was filtered off and the filtrate was evaporated to dryness obtaining a residue which was triturated with diisopropyl ether.

The precipitated white solid was collected and flash-chromatographed over silica gel (step gradient with methylene chloride-methanol 90:10, 85:15, 80:20) affording the title compound as a white solid (5.73 g, 53.3%), Rf = 0.20 (silica gel plates, eluent ethyl acetate-cyclohexane-acetic acid-water 10:10:2:1).
MS(FAB, 8-10kV, 2mA) m/z=934(MNa)⁺, 950(MK)⁺

### Preparation 13

HOOC(CH2)2C≡C-CH2
|
Fmoc-Ser(t-Bu)-Asn-Leu-Ser(t-Bu)-Thr(t-Bu)-NH-CH-COOCH3

The title compound was obtained by preparing separately the two reactants and directly condensing them without any intermediate purification.

### Reactant A

A solution of 3.18 g of Fmoc-Ser(t-Bu)-Asn-Leu-Ser(t-Bu)-Thr(t-Bu)-OH and 0.80 g of N-hydroxysuccinimide in 42 ml of anhydrous tetrahydrofuran was cooled to 0°C and 0.72 g of dicyclohexylcarbodiimide were added. The reaction was stirred overnight at room temperature and then diluted with 45 ml of anhydrous tetrahydrofuran. After filtration of the insoluble material the filtrate was directly used in the following condensation.

### Reactant B

1.47 g of (S)-2-(9-fluorenylmethyloxycarbonyl-amino)-4-octyne-1,8-dioic acid 1-methyl ester were dissolved in 38.2 ml of a 10% solution of diethylamine in anhydrous dimethylformamide at room temperature under magnetic stirring. After 30 min the solvent was evaporated to dryness and the residue was triturated with diethyl ether to afford 0.65 g of a white solid which was directly used in the following step.

### Condensation

Reactant B was added at room temperature under magnetic stirring to the solution A and stirring was continued overnight. After evaporation under vacuum of the reaction mixture, the residue was triturated with 320 ml of ethyl acetate to afford a white solid which was filtered off. The filtrate was evaporated to dryness and the residue was triturated with 50 ml of methylene chloride-methanol 95:5 to give an insoluble material which was filtered off again. The filtrate was evaporated to dryness and the residue was flash-chromatographed over silica gel (step gradient with methylene chloride-methanol 95:5, 90:10, 85:15, 80:20). Evaporation of the appropriate collected fractions afforded 1.75 g (49%) of the title compound as a white solid, Rf = 0.50 (silica gel plates, eluent chloroform-methanol-acetic acid-water 170:26:1:3).
MS(FAB, 8-10kV, 2mA) m/z=1093(MH)⁺, 870(MH-C₁₅H₁₁O₂)⁺

### Preparation 14

HOOC(CH2)2C≡C-CH2
|
H-Ser(t-Bu)-Asn-Leu-Ser(t-Bu)-Thr(t-Bu)-NH-CH-COOCH3

A solution of 1.71 g of HOOC-(CH2)2-C≡C-CH2
|
Fmoc-Ser(t-Bu)-Asn-Leu-Ser(t-Bu)-Thr(t-Bu)-NH-CH-COOCH3

in 22 ml of 8% diethylamine in anhydrous dimethylformamide was stirred at room temperature for 30 min. Evaporation to dryness gave a residue which was triturated twice with diethyl ether, affording the title compound as a white solid (1.37 g, 100%), Rf = 0.33 (silica gel plates, eluent butanol-acetic acid-water 4:1:1).
MS(FAB, 8-10kV, 2mA) m/z=892(MNa)⁺, 908(MK)⁺

### Preparation 15

CO-------CH2----------CH2——C≡C-----------CH2
|                                          |
Ser(t-Bu)-Asn-Leu-Ser(t-Bu)-Thr(t-Bu)-NH-CH-COOCH3

To a solution of 630 mg of 
A solution of 630 g of HOOC-(CH2)2-C≡C-CH2
H-Ser(t-Bu)-Asn-Leu-Ser(t-Bu)-Thr(t-Bu)-NH-CH-COOCH3
 in 665 ml of anhydrous dimethylformamide, N-methylmorpholine was added until pH=7.5 was reached. The solution was cooled to -5°C and 640 mg of benzotriazol-1-yl-oxytris(dimethylamino)phosphonium hexafluorophosphate (BOP) were added, and stirring was continued at -2°C for 72 hours.

After evaporation of the solvent, the residue was dissolved in 1 l of cold (5°C) ethyl acetate and washed successively with 3x400 ml of cold (5°C) 1N hydrochloric acid, 400 ml of brine, 2x400 ml of 5% sodium bicarbonate and 400 ml of brine. The organic phase was dried over anhydrous sodium sulfate and evaporated to dryness obtaining the title compound as a white solid (580 mg, 94%), Rf = 0.58 (silica gel plates, eluent butanol-acetic acid-water 4:1:1).
MS(FAB, 8-10kV, 2mA) m/z=874(MNa)⁺

### Preparation 16

CO---------CH2---------CH2——C≡C ---------CH2
|                                          |
Ser(t-Bu)-Asn-Leu-Ser(t-Bu)-Thr(t-Bu)-NH-CH-COOH

To a solution of 550 mg of 
CO-----------CH2-----------CH2---C≡C--------CH2
|                                          |
Ser(t-Bu)-Asn-Leu-Ser(t-Bu)-Thr(t-Bu)-NH-CH-COOCH3
 in 4 ml of methanol, 0.97 ml of 1N sodium hydroxide were added under magnetic stirring. After 2 hours 30 ml of water were added and methanol was evaporated. The aqueous solution was acidified to pH = 2 with 1N HCl and then extracted with 4x25 ml of ethyl acetate.

The organic phases were collected, washed with 2x50 ml of brine, dried over anhydrous sodium sulfate and evaporated to dryness, affording 420 mg (78%) of the title compound as a white solid, Rf = 0.47 (silica gel plates, eluent butanol-acetic acid-water 4:1:1).
MS(FAB, 8-10kV, 2mA) m/z=838(MH)⁺, 782(MH-C₄H₈)⁺, 726(MH-2C₄H₈)⁺

### Preparation 17

CO-------------CH2------CH2-- C≡C--------CH2
|                                        |
Ser(t-Bu)-Asn-Leu-Ser(t-Bu)-Thr(t-Bu)-NH-CH-CO-Val-Leu-Gly-OCH3

To a solution of 400 mg of 
CO---------CH2--------CH2---C≡C  --------CH2
|                                        |
Ser(t-Bu)-Asn-Leu-Ser(t-Bu)-Thr(t-Bu)-NH-CH-COOH
 in 40 ml of anydrous dimethylformamide were successively added 70 mg of N-hydroxybenzotriazole, 6 mg of 4-N,N-dimethylaminopyridine and 108 mg of dicyclohexylcarbodiimide. The solution was then stirred for 10 min at 0°C. A precooled solution (0°C) of 161 mg of H-Val-Leu-Gly-OCH₃.HCl (J.H. Jones et al; J. Chem. Soc,C, 1967p2371) and 0.052 ml of N-methylmorpholine in 13 ml of anhydrous dimethylformamide was then added. Stirring was continued at 0°C for 1 hour and at room temperature overnight. After evaporation of the solvent the residue was triturated with ethyl acetate and the solid material was filtered off. The filtrate was washed successively with 3x200 ml of 10% citric acid, 200 ml brine, 4x200 ml of 5% sodium bicarbonate, 2x200 ml brine.

The organic layer was dried over anhydrous sodium sulfate and evaporated to dryness, affording 405 mg (76%) of the title compound as a white solid, Rf = 0.38 (silica gel plates, eluent chloroform-methanol-acetic acid-water 170:26:1:3).
MS(FAB, 8-10kV, 2mA) m/z=1143(MNa)⁺, 1159(MK)⁺

### Preparation 18

CO------------CH2-----CH2--- C≡C --------CH2
|                                        |
Ser(t-Bu)-Asn-Leu-Ser(t-Bu)-Thr(t-Bu)-NH-CH-CO-Val-Leu-Gly-OH

To a solution of 370 mg of 
CO------------CH2-----CH2---C≡C----------CH2
|                                          |
Ser(t-Bu)-Asn-Leu-Ser(t-Bu)-Thr(t-Bu)-NH-CH-CO-Val-Leu-Gly-OCH3
 in 2 ml of methanol, 0.5 ml of 1N sodium hydroxide were added under magnetic stirring. After 2 hours at room temperature, the mixture was treated as described for Preparation 16, affording 350 mg of white solid. This product was purified by HPLC using RP-18 as stationary phase and eluent A [0.05% trifluoroacetic acid (TFA) in water] + eluent B (water-acetonitrile 3:7, 0.05% TFA) as mobile phase (gradient from 60 to 90% B in 40 min). The appropriate fractions were collected, 3 ml of dimethylformamide were added and the solvent was evaporated in vacuo. The oily residue was triturated with diethyl ether, affording the title compound (111 mg, 30%) as a white solid, Rf = 0.56 (silica gel plates, eluent butanol-acetic acid-water 4:1:1:).
MS(FAB, 8-10kV, 2mA) m/z=1129(MNa)⁺, 1145(MK)⁺

### Preparation 19

### H-Arg(Mtr)-Thr(t-Bu)-Asp(O-t-Bu)-Val-Gly-Ala-Gly-Thr(t-Bu)-Pro-NH₂

500 mg of Fmoc-Arg(Mtr)-Thr(t-Bu)-Asp(O-t-Bu)-Val-Gly--Ala-Gly-Thr(t-Bu)-Pro-NH₂ (obtained from Calbiochem-Novabiochem AG, Weidenmattweg 4, CH-4448 Laufelfingen, Switzerland) were dissolved in 3.5 ml of a 10% solution of diethylamine in dimethylformamide under magnetic stirring. After 60 min at room temperature, the solvent was evaporated to dryness. The residue was triturated with diethyl ether to afford 409 mg (99%), of the title compound, Rf = 0.24 (silica gel plates, eluent butanol-acetic acid-water 4:1:1).
MS(FAB, 8-10kV, 2mA) m/z=1253(MH)⁺, 1199(M-C₄H₈)⁺

### Preparation 20

### Fmoc-Lys(Boc)-Leu-Ser(t-Bu)-Gln-Glu(O-t-Bu)-Leu-His(Trt)--Lys(Boc)-Leu-Gln-Thr(t-Bu)-Tyr(t-Bu)-Pro-Arg(Mtr)-Thr(t --Bu)-Asp(O-t-Bu)-Val-Gly-Ala-Gly-Thr(t-Bu)-Pro-NH₂

To a solution of 709 mg of Fmoc-Lys(Boc)-Leu-Ser(t-Bu)-Gln-Glu-(O-t-Bu)-Leu-His(Trt)-Lys(Boc)-Leu-Gln-Thr(t-Bu)-Tyr(t-Bu)-Pro-OH (obtained from Calbiochem-Novabiochem, Switzerland) in 12 ml of anhydrous dimethylformamide, 42 mg of N-hydroxybenzotriazole, 65 mg of dicyclohexylcarbodiimide and 4 mg of 4-N,N-dimethylaminopyridine were added. After 10 min at 0°C, 360 mg of H-Arg(Mtr)-Thr(t-Bu)-Asp(O-t-Bu)-Val-Gly-Ala-Gly-Thr(t-Bu)-Pro-NH₂ dissolved in 6 ml of anhydrous dimethylformamide were added, and stirring was continued at room temperature overnight.

The solvent was removed and the residue was triturated with dichloromethane, filtered and washed on the filter several times with methylene chloride affording the crude title compound as a white solid which was used as such in the next step (1.01 g, 94%), Rf = 0.68 (silica gel plates, eluent butanol-acetic acid-water 4:1:1).
MS(FAB, 8-10kV, 2mA) m/z=3709(M+2H)⁺, 3466(M-C₁₉H₁₅)⁺

### Preparation 21

### H-Lys(Boc)-Leu-Ser(t-Bu)-Gln-Glu(O-t-Bu)-Leu-His(Trt)--Lys(Boc)-Leu-Gln-Thr(t-Bu)-Tyr(t-Bu)-Pro-Arg(Mtr)-Thr -(t-Bu)-Asp(O-t-Bu)-Val-Gly-Ala-Gly-Thr(t-Bu)-Pro-NH₂

350 mg of Fmoc-Lys(Boc)-Leu-Ser(t-Bu)-Gln-Glu(O-t-Bu)-Leu-His(Trt)-Lys(Boc)-Leu-Gln-Thr(t-Bu)-Tyr(t-Bu)-Pro-Arg(Mtr)-Thr(t-Bu)-Asp(O-t-Bu)-Val-Gly-Ala-Gly-Thr(t-Bu)-Pro-NH₂ were dissolved at room temperature under magnetic stirring in 4.2 ml of a 10% solution of diethylamine in anhydrous dimethylformamide. After 30 min the solvent was removed and the residue was triturated with diethyl ether. The precipitate was filtered and washed with diethyl ether, affording 326 mg of the impure title compound as a white solid, Rf = 0.60 (silica gel plates, eluent butanol-acetic acid-water 4:1:1).
MS(FAB, 8-10kV, 2mA) m/z=3485(M+2H)⁺, 3242(M-C₁₉H₁₅)⁺

### Preparation 22

### [S-(Z)]-2-(9-Fluorenylmethyloxycarbonylamino)-4-octene-1,8-dioic acid 1-methyl ester

Into a solution of 1.10 g of (S)-2-(9-fluorenylmethyloxycarbonylamino)-4- octyne-1,8-dioic acid 1-methyl ester in 50 ml of methanol, containing 0.25 g of 5% palladium on barium sulfate and 0.25 g of quinoline, hydrogen was bubbled at room temperature for 50 min. The catalyst was filtered off and the filtrate was evaporated to dryness obtaining a residue which was dissolved in ethyl acetate and washed twice with 10% citric acid and with brine to neutrality. The organic phase was dried over anhydrous sodium sulfate, filtered and the solvent removed, affording 1.05 g (96%) of the title compound as an oil. Rf = 0.75 (silica gel plate, eluent ethyl acetate-cyclohexane-acetic acid-water 10:10:2:1); [α]_{D}= + 11.9 (c=1, THF)

NMR (CDCl₃): δ_{H}= 8.50 (bs, 1H, COOH); 7.85-7.20 (m, 8H, ArH); 7.60 (bs, 1H, NH); 5.5 (m, 2H, CH=CH); 4.5-4 (m, 4H, >CH-CH₂-O-CONH-CH); 3.75 (s, 3H, COOCH₃); 2.6 (m, 2H, CH-CH₂); 2.40 (s, 4H, CH₂-CH₂).
MS (C.I. isobutane) m/z = 424 (MH)⁺.

### Preparation 23

H H
CH2C=C-(CH2)2-COOH
Fmoc-Ser(t-Bu)-Asn-Leu-Ser(t-Bu)-Thr(t-Bu)-NH-CH-COOCH3

The title compound was prepared as described in Preparation 13 starting from 2 g of Fmoc-Ser(t-Bu)-Asn-Leu-Ser(t-Bu)-Thr(t-Bu)-OH and from 1 g of (Z)-(S)-2-(9-fluorenylmethyloxycarbonylamino)-4-octene-1,8-dioic acid 1-methyl ester. Obtained: 0.94 g (70%). Rf= 0.57 (silica gel plate, eluent chloroform-methanol-water-acetic acid 170:26:3:1)
MS (FAB, 8-10 kV, 2mA) m/z= 1095 (MH)⁺, 1117 (MNa)⁺, 1133 (MK)⁺.

### Preparation 24

H H
CH2 C=C-(CH2)2-COOH
H-Ser(t-Bu)-Asn-Leu-Ser(t-Bu)-Thr(t-Bu)-NH-CH-COOCH3

The title compound was prepared as described in Preparation 14, starting from 0.9 g of 
H H
CH2 C=C-(CH2)2-COOH
Fmoc-Ser(t-Bu)-Asn-Leu-Ser(t-Bu)-Thr(t-Bu)-NH-CH-COOCH3
 and obtaining 0.71 g (100%). Rf= 0.41 (silica gel plate, eluent butanol-acetic acid-water 4:1:1)
MS (FAB, 8-10 kV, 2mA) m/z= 873 (MH)⁺, 895 (MNa)⁺, 911 (MK)⁺

### Preparation 25

H H
CO------------CH2------CH2--- C=C---------CH2
Ser(t-Bu)-Asn-Leu-Ser(t-Bu)-Thr(t-Bu)-NH-CH-COOCH3

The title compound was prepared as described in Preparation 15, starting from 0.72 g of 
H H
HOOC--(CH2)2—C=C—CH2
H-Ser(t-Bu)-Asn-Leu-Ser(t-Bu)-Thr(t-Bu)-NH-CH-COOCH3
 and obtaining: 0.46 g (66%). Rf = 0.62 (silica gel plate, eluent butanolacetic acid-water 4:1:1)
MS (FAB, 8-10 kV, 2mA) m/z= 855 (MH)⁺, 877 (M+Na)⁺, 799 (MH-C₄H₈)⁺, 742 (MH-2C₄H₈)⁺, 686 (MH-3C₄H₈)⁺.

### Preparation 26

H H
CO CH2 CH2 C=C CH2
Ser(t-Bu)-Asn-Leu-Ser(t-Bu)-Thr(t-Bu)-NH-CH-COOH

The title compound was prepared as described in Preparation 16, starting from 0.43 g of 
H H
CO------------CH2------CH2---- C=C---------CH2
Ser(t-Bu)-Asn-Leu-Ser(t-Bu)-Thr(t-Bu)-NH-CH-COOCH3
 and obtaining 0.42 g (100%). Rf= 0.47 (silica gel plate, eluent butanol-acetic acid-water 4:1:1)
MS (FAB, 8-10 kV, 2mA) m/z= 841 (MH)⁺, 672 (MH-3C₄H₈)⁺

### Preparation 27

H H
CO------------CH2------CH2---- C=C---------CH2
Ser(t-Bu)-Asn-Leu-Ser(t-Bu)-Thr(t-Bu)-NH-CH-CO-Val-Leu-Gly-OCH3

The title compound was prepared as described in Preparation 17 starting from 0.42 g of 
H H
CO--------------CH2------CH2---- C=C-------CH2
Ser(t-Bu)-Asn-Leu-Ser(t-Bu)-Thr(t-Bu)-NH-CH-COOH
 and from 0.17 g of H-Val-Leu-Gly-OCH₃.HCl.

Obtained: 0.50 g (8%). Rf = 0.33 (silica gel plate, eluent chloroform-methanol-water-acetic acid 170:26:3:1)
MS (FAB, 8-10 kV, 2mA) m/z = 1145 (MNa)⁺, 1034 (MH-GlyOCH₃)⁺, 921 (MH-LeuGlyOCH₃)⁺.

### Preparation 28

H H
CO------------CH2------CH2---- C=C---------CH2
Ser(t-Bu)-Asn-Leu-Ser(t-Bu)-Thr(t-Bu)-NH-CH-CO-Val-Leu-Gly-OH

The title compound was prepared as described in Preparation 18 starting from 0.48 g of 
H H
CO------------CH2-------CH2——C=C------CH2
Ser(t-Bu)-Asn-Leu-Ser(t-Bu)-Thr(t-Bu)-NH-CH-CO-Val-Leu-Gly-OCH3
 and obtaining 0.29 g (60.8%). Rf = 0.55 (silica gel plates, eluent butanol-acetic acid-water 4:1:1)
MS (FAB, 8-10 kV, 2mA) m/z = 1131 (MNa)⁺, 1034 (MH-GlyOH)⁺, 921(MH-LeuGlyOH)⁺.

### Preparation 29

### 4-(2-Tetrahydropyranyloxy)butanol

A mixture of 70 g of 3,4-dihydro-2H-pyran and 600 g of 1,4-butanediol was stirred for 30 minutes, then 0.6 g of p-toluenesulfonic acid monohydrate were added. Stirring was continued for 3 hours.

The mixture was diluted with 600 ml of water and extracted three times with diethyl ether (500 ml each). The organic phases were collected and dried over sodium sulfate and evaporated to dryness to give 105 g of crude title compound as an oil. Distillation of the residue under vacuum afforded 86 g (59%) of the title compound as a colourless oil, b.p. 82-84°C (0.3 mmHg).

H-NMR (CDCl₃): δ_{H} = 4.65 (m, 1H, O-CH-O); 4.10 - 3.35 (m, 6H, 2 CH₂O and C**H**₂OH); 2.15 (bs, 1H, OH); 2.0 - 1.4 (m, 1OH, CH-C**H**₂-C**H**₂-C**H**₂ and C**H**₂-C**H**₂-CH₂OH)
MS(E.I. 20 eV) m/z = 173(M-H)⁺, 155 (M-H₂O)⁺

### Preparation 30

### 4-(2-Tetrahydropyranyloxy)butanal

To a suspension of 148.32 g of pyridinium chlorochromate and 15 g of anhydrous sodium acetate in 800 ml of dichloromethane, a solution of 80 g of 4-(2-tetrahydropyranyloxy)butanol in 80 ml of dichloromethane was added.

Stirring was continued for 2 hours then 800 ml of diethyl ether were added, the surnatant was decanted and filtered through 350 g of Florisil. The solvent was removed under vacuum to give 62 g of a yellow oily residue. Distillation of the residue under vacuum afforded 39.5 g (50%) of the title compound as a colourless oil, b.p. 70-72°C (0.1 mmHg).

H-NMR(CDCl₃): δ_{H} = 9.72 (t, J=lHz, 1H, CHO); 4.60 (m, 1H, OCHO); 4.00 - 3.25 (m, 4H, 2CH₂O); 2.55 (dt, J=6.5, 1 Hz, CH₂CHO); 2.15 - 1.70 (m, 2H, CH₂C**H**₂CHO); 1.70 - 1.35 (m, 6H, CH₂CH₂CH₂)
MS(E.I. 70 eV) m/z = 172 (M)⁺, 143 (M-CHO)⁺

### Preparation 31

### (E)-6-(2-Tetrahydropyranyloxy)-2-hexenal

A mixture of 20 g of 4-(2-tetrahydropyranyloxy)butanal and 35.3 g of (formylmethylene)triphenylphosphorane in 300 ml of toluene was refluxed for 7 hours in a nitrogen atmosphere. After cooling, the solvent was removed under vacuum, the residue was treated with 200 ml of hexane and the insoluble material was filtered off. The filtrate was then treated with charcoal, filtered on a Celite pad and evaporated to dryness to give 18.2 (79%) of the title compound as a yellow oil, b.p. 92-95°C (0.1 mbar)

H-NMR(CDCl₃): δ_{H} = 9.55(d, J= 7.5 Hz, 1H, CHO); 6.93(dt, J=15, 6 Hz, =C**H**-CH₂); 6.20(ddt, J=15, 7.5, 1 Hz, 1H,=C**H**-CHO); 4.6 (m, 1H, O-CH-O); 4.0-3.25(m, 4H, 2CH₂O); 2.65-2.35(m, 2H, C**H**₂-CH=); 2.0-1.5 (m, 8H, C**H**₂CH₂CH= and C**H**₂C**H**₂C**H**₂)
MS(E.I. 70 eV) m/z = 197 (M-H)⁺, 130 (M-C₄H₄O)⁺

### Preparation 32

### (E)-6-(2-Tetrahydropyranyloxy)-2-hexenol

To an ice cold solution of 18 g of (E)-6-(2-tetrahydropyranyloxy)-2-hexenal in 90 ml of water and 160 ml of methanol, 0.85 g of sodium borohydride were added. Stirring was continued for 15 min, the methanol was removed under vacuum and the aqueous solution was extracted twice with ethyl acetate. The organic phase was dried over anhydrous sodium sulfate and evaporated to dryness to give 15 g (83.2%) of the title compound, as a colourless oil.

H-NMR (CDCl₃): δ_{H} = 5.72 (m, 2H, CH=CH); 4.57 (m, 1H, OCHO); 4.1 (m, 2H, C**H**₂OH); 4.00-3.25 (m, 4H, 2CH₂O); 2.40-2.00 (m, 2H, C**H**₂-CH=); 2.00-1.25 (m, 8H, C**H**₂C**H**₂C**H**₂ and O-CH₂C**H**₂)
MS(E.I. 70 eV) m/z = 101 (M-C₆H₁₁O)⁺

### Preparation 33

### (E)-2-(6-Bromo-4-hexenoxy)tetrahydropyran

A solution of 22 g of (E)-6-(2-tetrahydropyranyloxy)-2-hexenol and 36.5 g of carbon tetrabromide in 220 ml of dichloromethane was chilled to -55°C under nitrogen and 28.4 g of triphenylphosphine were added portionwise. Stirring was continued at the same temperature for 5 minutes, then the temperature was allowed to rise to 3°C. 150 ml of 7% aqueous NaHCO₃ were added, the organic phase was collected, dried over anhydrous sodium sulfate and evaporated to dryness to give 22 g as a crude oil which was chromatographed over silica gel (ethyl acetate-hexane 20:80) affording 11 g (38%) of the title compound, as a colourless oil.

H-NMR (CDCl₃): δ_{H} = 5.75 (m, 2H, CH=CH); 4.60 (m, 1H, OCHO); 3.95 (d, J = 5Hz, 2H CH₂Br); 4.0-3.25 (m, 4H, 2OCH₂); 2.20 (m, 2H, C**H**₂-CH=); 2.0-1.35(m, 8H, C**H**₂C**H**₂C**H**₂ and C**H**₂CH₂CH=)
MS(E.I. 70 eV) m/z = 262,264 (M)⁺, 182 (M-Br)⁺

### Preparation 34

### [S-[R*,S*-(E)]]-2-Isopropyl-2,5-dihydro-3,6-dimethoxy-5-[6-(2-tetrahydropyranyloxy)-2-hexenyl]pyrazine

Using the same procedure of as Preparation 4, starting from 8.5 g of 2-(6-bromo-4-hexenoxy)tetrahydropyran and 5.4 g of (R)-(-)-2,5-dihydro-3,6-dimethoxy-2-isopropylpyrazine, 9 g (84%) of the title compound were obtained as an oil. [α]_{D}= ⁺14.5 (c= 1, CH₂Cl₂)

H-NMR (CDCl₃): δ_{H} = 5.40 (m, 2H, CH=CH); 4.57 (m, 1H, OCHO); 4.2-3.25 (m, 6H, 2OCH₂ and 2=N-CH); 3.70(s, 6H, 2OCH₃); 2.6-1.90 (m, 5H, C**H**₂-CH=CH-C**H**₂ and (CH₃)₂C**H**); 1.90-1.30 (m, 8H, C**H**₂C**H**₂C**H**₂ and C**H**₂CH₂CH=); 1.05 and 0.70 (d, J=7Hz, gem-CH₃),
MS(E.I. 70 eV) m/z = 366 (M)⁺, 323 (M-C₃H₇)⁺

### Preparation 35

### [S-(E)]-2-(9-Fluorenylmethyloxycarbonylamino)-4-octene-1,8-dioic acid 1-methyl ester

Using the same procedure as Preparation 5, starting from 9 g of [S-[R*,S*-(E)]]-2-isopropyl-2,5-dihydro-3,6-dimethoxy-5-[6-(2-tetrahydropyranyloxy)-2-hexenyl]pyrazine, 4.1 g of the title compound were obtained, as a white powder, m.p. 95-97°C, [α]_{D}= +12.6 (c=1, THF)

H-NMR (CDCl₃): δ_{H} = 8.5 (bs, 1H, COOH); 7.90-7.10 (m, 9H, Fmoc and NH) 5.75-5.0 (m, J_{AB}= 15 Hz, 2H, CH=CH); 4.8 (m, 4H, NHC**H**COO and C**H**C**H**₂OCO); 3.7 (s, 3H, COOCH₃); 2.7-2.1 (m, 6H, C**H**₂CH= CHC**H**₂C**H**₂)
MS (FAB⁺, 8-10 kV, 2mA): m/z = 424 (MH)⁺, 446 (M+Na)⁺

### Preparation 36

H
CH2C=C-(CH2)2-COOH
H
Fmoc-Ser(t-Bu)-Asn-Leu-Ser(t-Bu)-Thr(t-Bu)-NH-CH-COOCH3

The title compound was prepared as described in Preparation 13 starting from 2.74 g of Fmoc-Ser(t-Bu)-Asn-LeuSer(t-Bu)-Thr(t-Bu)-OH and from 1.5 g of (E)-(S)-2-(9-fluorenylmethyloxycarbonylamino)-4-octene-1,8-dioic acid 1-methyl ester. Obtained 1.57 g (50%). Rf=0.60 (silica gel plate, eluent chloroform-methanol-water-acetic acid 170:26:3:1)
MS (FAB, 8-10 kV, 2mA) m/z = 1095 (MH)⁺, 983 (MH-2C₄H₈)⁺

### Preparation 37

H
HOOC-(CH2)2-C=C-CH2
H
H-Ser(t-Bu)-Asn-Leu-Ser(t-Bu)-Thr(t-Bu)-NH-CH-COOCH3

H
HOOC-(CH2)2-C=C-CH2
H
0.95 gof Fmoc-Ser(t-Bu)-Asn-Leu-Ser(t-Bu)-Thr(t-Bu)-NH-CH-COOCH3

were treated as described in Preparation 14, affording the title compound as a white solid (0.76 g, 100%), Rf= 0.43 (silica gel plates, eluent butanol-acetic acid-water 4:1:1)
MS (FAB, 8-10 kV, 2mA) m/z = 873 (MH)⁺, 911 (MK)⁺, 817 (MH-C₄H₈)⁺, 761 (MH-2C₄H₈)⁺, 704 (MH-3C₄H₈)⁺

### Preparation 38

H
CO------------CH2-----CH2———C=C---------CH2
H
Ser(t-Bu)-Asn-Leu-Ser(t-Bu)-Thr(t-Bu)-NH-CH-CO-COOCH3

H
HOOC-(CH2)2-C=C-CH2
H
0.7 g of H-Ser(t-Bu)-Asn-Leu-Ser(t-Bu)-Thr(t-Bu)-NH-CH-COOCH3

were treated as described in Preparation 15, affording the title compound as a white solid (0.5 g, 73%), Rf= 0.61 (silica gel plates, eluent: butanol-acetic acid-water 4:1:1).
MS (FAB, 8-10kV, 2mA) m/z = 855 (MH)⁺, 877 (MNa)⁺, 799 (MH-C₄H₈)⁺, 742 (MH-2C₄H₈)⁺, 686 (MH-3C₄H₈)⁺

### Preparation 39

H
CO------------CH2------CH2----C=C---------CH2
H
Ser(t-Bu)-Asn-Leu-Ser(t-Bu)-Thr(t-Bu)-NH-CH-COOH

H
|
CO----------CH2-----CH2—C=C-------------CH2
|                        |              |
|                        H              |
0.45 g of Ser(t-Bu)-Asn-Leu-Ser(t-Bu)-Thr(t-Bu)-NH-CH-COOCH3

were treated as described in Preparation 16, giving the title compound as a white solid (0.44, 100%), Rf= 0.45 (silica gel plates, eluent butanol-acetic acid-water 4:1:1).
MS (FAB, 8-10 kV, 2mA) m/z= 841 (MH)⁺, 785 (MH-C₄H₈)⁺, 728 (MH-2C₄H₈)⁺, 672 (MH-3C₄H₈)⁺

### Preparation 40

H
CO------------CH2-----CH2———C=C ---------CH2
H
Ser(t-Bu)-Asn-Leu-Ser(t-Bu)-Thr(t-Bu)-NH-CH-CO-Val-Leu-Gly-OCH3

H
CO------------CH2-----CH2-- C=C---------------CH2
H
0.42 g of Ser(t-Bu)-Asn-Leu-Ser(t-Bu)-Thr(t-Bu)-NH-CH-COOH

and 0.17 g of H-Val-Leu-Gly-OCH₃.HCl were treated as described in Preparation 17, affording 0.56 g (100%) of the title compound, Rf=0.78 (silica gel plates, eluent butanol-acetic acid-water 4:1:1).
MS (FAB, 8-10 kV, 2mA) m/z= 1124 (MH)⁺, 1035 (MH-GlyOCH₃)⁺, 922 (MH - LeuGlyOCH₃)⁺

### Preparation 41

H
CO------------CH2-----CH2——C=C--------CH2
H
Ser(t-Bu)-Asn-Leu-Ser(t-Bu)-Thr(t-Bu)-NH-CH-CO-Val-Leu-Gly-OH

H
CO------------CH2-----CH2——C=C--------CH2
H
0.52 g of Ser(t-Bu)-Asn-Leu-Ser(t-Bu)-Thr(t-Bu)-NH-CH-CO-Val-Leu-

-Gly-OCH₃ were treated as described in Preparation 18, affording 0.45 g of title compound as a crude white solid. This was purified by HPLC using RP-18 as stationary phase, eluent A (0.05% TFA in water) + eluent B (water-acetonitrile 3:7, 0.05% TFA) as mobile phase (gradient 30 to 70%B in 20 min).

The appropriate fractions were collected, the acetonitrile was evaporated in vacuo and the remaining solution was lyophilized, affording the title compound (98 mg, 19%) as a white solid, Rf= 0.48 (silica gel plates, eluent butanol-acetic acid-water 4:1:1).
MS (FAB, 8-10 kV, 2mA) m/z= 1132 (MNa)⁺, 923 (MNa - Leu GlyOH)⁺

### Preparation 42

### Fmoc-Arg(Mtr)-Thr(t-Bu)-OBzl

A) 0.80 g of Fmoc-Thr(t-Bu)-OBzl were treated as described in Preparation 7 - part A.
B) 1 g of Fmoc-Arg(Mtr)-OH and the solution A were treated as described in Preparation 7 - Part B. After the usual acid-base washings, the organic phase was dried over anhydrous sodium sulfate. After filtration and evaporation to dryness, the residue was triturated with hexane obtaining a white solid (1.34 g, 94%), Rf = 0.66 (silica gel plates, eluent ethyl acetate).
   MS (FAB, 8kV, 2mA) m/z = 857(MH)⁺, 801(MH-C₄H₈)⁺.

### Preparation 43

### Fmoc-Arg (Mtr)-Thr (t-Bu)-OH

1.28 g of Fmoc-Arg(Mtr)-Thr(t-Bu)-OBzl were dissolved in a mixture of 25 ml of methanol and 5 ml dimethylformamide.

0.09 ml of acetic acid and 160 mg of palladium on charcoal were added and hydrogen was bubbled for 3 hours into the solution at 40°C. The catalyst was then filtered off and the filtrate evaporated to dryness. The residue was triturated with diethyl ether, obtaining a white precipitate (0.84 g, 73%), Rf = 0.35 (silica gel plates, eluent cyclohexane-ethyl acetate-acetic acid-water 10:10:2:1).
MS (FAB, 8kV, 2mA) m/z = 789(MNa)⁺, 767(MH)⁺, 711(MH-C₄H₈)⁺.

### Preparation 44

### Fmoc-Gly-Ala-OH

3 g of L-alanine were dissolved in 30 ml of dimethylformamide and 33.6 ml of 1N sodium hydroxide were added. The solution was evaporated to dryness, redissolved in dimethylformamide and evaporated. This operation was repeated twice. The obtained oil was triturated with diethyl ether and a white solid was obtained. This was redissolved in tetrahydrofuran (200 ml) and treated with 13.26 g of N-fluorenylmethoxycarbonylglycine N-hydroxysuccinimidyl ester (Fmoc-Gly-OSu). After stirring for 2 days at room temperature, 200 ml of water were added, the organic solvent evaporated under vacuum and 10% hydrochloric acid was added until pH=2. The aqueous solution was extracted with 3 x 150 ml of ethyl acetate. The three collected organic fractions were washed with 2 x 300 ml of brine and dried. After evaporation of the solvent the obtained residue was flash-chromatographed over silica-gel (step gradient with ethyl acetate, ethyl acetate-methanolacetic acid 99:1:0.1, 98:2:0.1, 95:5:0.1, 85:15:0.1, 70:30:0.1). The appropriate fractions were collected and the solvent evaporated, obtaining an oily residue which, after trituration with a mixture of diethyl ether-hexane, afforded 5.8 g (47%) of the title compound, Rf = 0.34 (silica gel plates, eluent cyclohexane-ethyl acetate-acetic acid-water 10:10:2:1).
MS (FAB, 8kV, 2mA) m/z = 391(MNa)⁺, 369(MH)⁺, 351(MH-H₂O)⁺.

### Preparation 45

### Fmoc-Gly-Ala-Gly-O-t-Bu

3.71 g of Fmoc-Gly-Ala-OH and 1.68 g of HCl·H-Gly-O-t-Bu were condensed using the method described in Preparation 17 and using tetrahydrofuran as solvent. After recrystallization with ethyl acetate, 2.56 g (53%) of the title compound were obtained as a white powder, Rf = 0.44 (silica gel plates, eluent cyclohexane-ethyl acetate-acetic acid-water 10:10:2:1).
MS (FAB, 8kV, 2mA) m/z = 482(MH)⁺, 426(MH-C₄H₈)⁺.

### Preparation 46

### Z-Val-Gly-Ala-Gly-O-t-Bu

2.4 g of Fmoc-Gly-Ala-Gly-O-t-Bu were deprotected with 10% diethylamine in dimethylformamide, obtaining an oily residue (H-Gly-Ala-Gly-O-t-Bu). This was then condensed with 1.46 g of Z-Val-OH as described in Preparation 7-part B. The title compound was obtained as a white solid (1.86 g, 65.3%), Rf = 0.43 (silica gel plates, eluent cyclohexane-ethyl acetate-acetic acid-water 10:10:2:1).
MS (FAB, 8kV, 2mA) m/z = 515(MNa)⁺, 493(MH)⁺, 437(MH-C₄H₈)⁺.

### Preparation 47

### H-Val-Gly-Ala-Gly-O-t-Bu

A solution of 1.81 g of Z-Val-Gly-Ala-Gly-O-t-Bu in 25 ml methanol, was treated with 180 mg of palladium on charcoal 10% and hydrogen was bubbled at room temperature. After 50 min the catalyst was removed by filtration and the solvent evaporated. Trituration of the residue with diethyl ether afforded 1.71 g (86%) of the title compound as a white solid, Rf = 0.39 (silica gel plates, eluent dichloromethane-methanol 8:2).
MS (FAB, 8kV, 2mA) m/z = 381(MNa)⁺, 359(MH)⁺, 303(MH-C₄H₈)⁺.

### Preparation 48

### Z-Ala-Val-Gly-Ala-Gly-O-t-Bu

0.99 g of Z-Ala-OH and 1.17 g of H-Val-Gly-Ala-Gly-O-t-Bu were treated as described in Preparation 7-Part B .After final trituration with diethyl ether, 1.44 g (57.9%) of the title compound were obtained, Rf = 0.73 (silica gel plates, eluent dichloromethane-methanol 8:2).
MS (FAB, 8kV, 2mA) m/z = 586(MNa)⁺, 564(MH)⁺, 508(MH-C₄H₈)⁺.

### Preparation 49

### Z-Ala-Val-Gly-Ala-Gly-OH

A solution of 1.39 g of Z-Ala-Val-Gly-Ala-Gly-O-t-Bu in 14 ml of trifluoroacetic acid was stirred at 0°C for 80 min. The solution was evaporated to dryness and the residue was redissolved in dimethylformamide and evaporated twice. The obtained oily residue was triturated with diethyl ether, affording the title compound as a white solid (1.24 g, 100%), Rf = 0.35 (silica gel plates, eluent butanol-acetic acid-water 4:1:1).
MS (FAB, 8kV, 2mA) m/z = 546(MK)⁺, 530(MNa)⁺, 508(MH)⁺.

### Preparation 50

### Fmoc-Thr(t-Bu)-Pro-NH₂

5.00 g of Fmoc-Thr(t-Bu)-OH, 1.89 g of H-Pro-NH₂·HCl and 1.38 ml of N-methylmorpholine were dissolved in a mixture of 50 ml of methylene chloride and 80 ml of dimethylformamide. The solution was chilled in an ice-bath and 2.85 g dicyclohexylcarbodiimide and 154 mg of 4-N,N-dimethylaminopyridine were added. After 1 hour at 0°C and 5 hours at room temperature, the solution was filtered and evaporated to dryness. The residue was diluted with ethyl acetate and the usual acidic and basic washings were performed. After anhydrification of the organic layer the solvent was removed and the residue was recristallized with a mixture of hot ethyl acetate and hexane affording 5.5 g (88%) of the title compound, Rf = 0.47 (silica gel plates, eluent dichloromethane-methanol 9:1).
MS (FAB, 8kV, 2mA) m/z = 494(MH)⁺, 438(MH-C₄H₈)⁺.

### Preparation 51

### Z-Ala-Val-Gly-Ala-Gly-Thr(t-Bu)-Pro-NH₂

527 mg of Fmoc-Thr(t-Bu)-Pro-NH₂ were deprotected with 10% diethylamine in dimethylformamide, obtaining an oily residue [H-Thr(t-Bu)-Pro-NH₂]. This was condensed with 600 mg of Z-Ala-Val-Gly-Ala-Gly-OH as described in Preparation 7-pan B. The obtained crude product was flash-chromatographed on silica gel (step gradient with methylene chloride-methanol 9:1, 85:15, 8:2), affording 247 mg (30%) of the title compound, Rf = 0.43 (silica gel plates, eluent dichloromethane-methanol 85:15).
MS (FAB, 8kV, 2mA) m/z = 784(MNa)⁺, 762(MH)⁺, 705(MH-C₄H₈)⁺.

### Preparation 52

### H-Ala-Val-Gly-Ala-Gly-Thr(t-Bu)-Pro-NH₂

A solution of 415 mg of Z-Ala-Val-Gly-Ala-Gly-Thr(t-Bu)-Pro-NH₂ in 30 ml of methanol and 3 ml dimethylformamide, was treated as described in Preparation 47, obtaining 324 mg (94%) of the title compound , Rf = 0.59 (silica gel plates, eluent chloroform-methanol-32% ammonia 15:5:1).
MS (FAB, 8kV, 2mA) m/z = 649(MNa)⁺, 627MH)⁺, 571(MH-C₄H₈)⁺.

### Preparation 53

### Fmoc-Arg(Mtr)-Thr(t-Bu)-Ala-Val-Gly-Ala-Giy-Thr(t-Bu)-Pro-NH₂

366 mg of Fmoc-Arg(Mtr)-Thr(t-Bu)-OH and 300 mg of H-Ala-Val-Gly-Ala-Gly-Thr(t-Bu)-Pro-NH₂ were condensed as described in Preparation 7-part B. The crude product was then purified by flash-chromatography (step gradient with methylene chloride:methanol 9:1, 85:15). Obtained: 425 mg (68.8%) of the title compound, Rf = 0.66 (silica gel plates, eluent dichloromethane-methanol 85:15).
MS (FAB, 8kV, 2mA) m/z = 1396(MNa)⁺, 1357(MH)⁺, 1318(MH-C₄H₈)⁺.

### Preparation 54

### H-Arg-(Mtr)-Thr(t-Bu)-Ala-Val-Gly-Ala-Gly-Th r(t-Bu)-Pro-NH₂

430 mg of Fmoc-Arg(Mtr)-Thr(t-Bu)-Ala-Val-Gly-Ala-Gly-Thr(t-Bu)-Pro-NH₂ were treated as described in Preparation 19. Obtained 363 mg (100%), Rf = 0.66 (silica gel plates, eluent dichloromethane-methanol 8:2).
MS (FAB, 8kV, 2mA) m/z = 1174(MNa)⁺, 1152(MH)⁺, 1096(MH-C₄H₈)⁺.

### Preparation 55

### Fmoc-Lys(Boc)-Leu-Ser(t-Bu)-Gln-Glu(O-t-Bu)-Leu-His(Trt)-Lys(Boc)-Leu-Gln-Thr(t-Bu)-Tyr(t-Bu)-Pro-A rg(Mtr)-Thr(t-Bu)-Ala-Val-Gly-Ala-Gly-Thr(t-Bu)-Pro-NH₂

772 mg of Fmoc-Lys(Boc)-Leu-Ser(t-Bu)-Gln-Glu(O-t-Bu)-Leu-His(Trt)-Lys(Boc)-Leu-Gln-Thr(t-Bu)-Tyr(t-Bu)-Pro-OH and 359 mg of H-Arg(Mtr)-Thr(t-Bu)-Asp(O-t-Bu)-Ala-Val-Gly-Ala-Gly-Thr(t-Bu)-Pro-NH₂ were treated as described in Preparation 24. Obtained: 1.11 g (98%). Rf = 0.57 (silica gel plates, eluent butanol-acetic acid-water 4:1:1).
MS (FAB, 8kV, 2mA) m/z = 3608(MH)⁺.

### Preparation 56

### H-Lys(Boc)-Leu-Ser(t-Bu)-Gln-Glu(O-t-Bu)-Leu-His(Trt)-Lys(Boc)-Leu-Gln-Thr(t-Bu)-Tyr(t-Bu)-Pro-Arg(Mtr)-Thr(t-Bu)-Ala-Val-Gly-Ala-Gly-Thr(t-Bu)-Pro-NH₂

531 mg of Fmoc-Lys(Boc)-Leu-Ser(t-Bu)-Gln-Glu(O-t-Bu)-Leu-His-(Trt)-Lys(Boc)-Leu-Gln-Thr(t-Bu)-Tyr(t-Bu)-Pro-Arg(Mtr)-Thr(t-Bu)-Ala-Val-Gly-Ala-Gly-Thr(t-Bu)-Pro-NH₂ were treated as described in Preparation 21. Obtained: 506 mg, (100%), Rf = 0.50 (silica gel plates, eluent butanol-acetic acid-water 4:1:1).
MS (FAB, 8kV, 2mA) m/z = 3410(MNa)⁺.

### EXAMPLE 1

### Step (a)

CO------------CH2-----CH2--- C≡C ---------CH2
|                                          |
Ser(t-Bu)-Asn-Leu-Ser(t-Bu)-Thr(t-Bu)-NH-CH-CO-Val-Leu
-Gly-Lys(Boc)-Leu-Ser(t-Bu)-Gln-Glu(O-t-Bu)-Leu-His(Trt)-
-Lys(Boc)-Leu-Gln-Thr(t-Bu)-Tyr(t-Bu)-Pro-Arg(Mtr)-Thr(t-Bu)-
-Asp(O-t-Bu)-Val-Gly-Ala-Gly-Thr(t-Bu)-Pro-NH2

To a solution of 80 mg of 
CO------------CH2-----CH2--- C≡C --------CH2
|                                        |
Ser(t-Bu)-Asn-Leu-Ser(t-Bu)-Thr(t-Bu)-NH-CH-CO-Val-Leu-Gly-OH
 in 3.8 ml of anhydrous dimethylformamide 10.7 mg of N-hydroxybenzotriazole, 18.6 mg of dicyclohexylcarbodiimide and 1 mg of 4-N,N-dimethylaminopyridine were added successively. After 10 min at 0°C, 255 mg of H-Lys(Boc)-Leu-Ser(t-Bu)--Gln-Glu(O-t-Bu)-Leu-His(Trt)-Lys(Boc)-Leu-Gln--Thr(t-Bu)-Tyr(t-Bu)-Pro-Arg(Mtr)-Thr(t-Bu)-Asp(O-t-Bu)-Val-Gly-Ala-Gly-Thr(t-Bu)-Pro-NH₂ were added and stirring continued at 0°C for 1 hour and overnight at room temperature. After evaporation of the solvent, 6 ml of methylene chloride were added to the residue and stirring was continued for 2 hours. The gelatinous precipitate was filtered and washed successively with methylene chloride and diethyl ether. The obtained title compound (260 mg) was used as such in the next step, Rf = 0.62 (silica gel plates, eluent butanol-acetic acid-water 4:1:1).
MS(FAB, 8-10kV, 2mA) m/z=4574(M2H)⁺, 4332(MH-C₁₉H₁₅)+

### Step (b)

CO——CH2—CH2—C≡C —CH2
|                                    |
Ser-Asn-Leu-Ser-Thr-NH-CH-CO-Val-Leu-Gly-Lys-Leu-Ser-Gln-
-Glu-Leu-His-Lys-Leu-Gln-Thr-Tyr-Pro-Arg-Thr-Asp-Val-Gly-
-Ala-Gly-Thr-Pro-NH2.4TFA

240 mg of 
CO-------------CH2-----CH2--- C≡C --------CH2
|                                        |
Ser(t-Bu)-Asn-Leu-Ser(t-Bu)-Thr(t-Bu)-NH-CH-CO-Val-Leu
-Gly-Lys(Boc)-Leu-Ser(t-Bu)-Gln-Glu(O-t-Bu)-Leu-His(Trt)-Lys-Boc)-Leu-Gln-Thr(t-
Bu)-Tyr(t-Bu)-Pro-Arg(Mtr)-Thr(t-Bu)-Asp(O-t-Bu)-Val-Gly-Ala-Gly-Thr(t-Bu)-Pro-NH2
 were dissolved at 0°C under magnetic stirring in a mixture of TFA thioanisole-trimethylsilyl bromide-m-cresole 25.3:3.8:4.2:0.4. After 45 min the reaction mixture was evaporated and the residue was triturated with diethyl ether. The precipitate was filtered, washed with diethyl ether and then dissolved at 0°C under magnetic stirring in a mixture of TFA-phenol-methyl ethyl sulfide 22.2:5.8:5.8. The reaction mixture was kept at 0°C for 60 min under a nitrogen atmosphere, then was evaporated to small volume and the residue was triturated with diethyl ether, affording a white precipitate which was collected by filtration and dried. The crude product was purified by HPLC using RP-18 as stationary phase and eluent A (0.05% TFA in water) + eluent B (water-acetonitrile 3:7 + 0.05% TFA) (gradient 60 to 90% B in 40 min). The appropriate fractions were collected, the organic solvents were evaporated in vacuo and the remaining aqueous solution was freeze-dried, obtaining 22 mg of the title compound as a liophyle. MS(FAB, 8-10kV, 2mA) m/z= 3359 (M2H)⁺

| **Aminoacid analysis** | | | | | |
|---|---|---|---|---|---|
| Arg | 1.03 | (1) | Leu | 4.45 | (5) |
| Ala | 1.02 | (1) | Lys | 1.92 | (2) |
| Asp | 1.96 | (2) | Pro | 2.00 | (2) |
| Glu | 2.99 | (3) | Ser | 2.89 | (3) |
| Gly | 2.90 | (3) | Tyr | 1 | |
| His | 0.95 | (1) | Thr | 3.85 | (4) |
| | | | Val | 1.69 | (2) |

### EXAMPLE 2

### Step (a)

H H
CO------------CH2-----CH2——C=C——————————CH2
Ser(t-Bu)-Asn-Leu-Ser(t-Bu)-Thr(t-Bu)-NH-CH-CO-Val-Leu
-Gly-Lys(Boc)-Leu-Ser(t-Bu)-Gln-Glu(O-t-Bu)-Leu-His(Trt)-Lys(Boc)-Leu-Gln-
Thr(t-Bu)-Tyr(t-Bu)-Pro-Arg(Mtr)-Thr(t-Bu)-Asp(O-t-Bu)-Val-Gly-Ala-Gly-Thr(t-
Bu)-ProNH2

The title compound was prepared as described in Example 1(a) starting from 0.15 g of 
H H

CO------------CH2-----CH2 — C=C ---------CH2

Ser(t-Bu)-Asn-Leu-Ser(t-Bu)-Thr(t-Bu)-NH-CH-CO-Val-Leu
-Gly-OH and from 0.47 g of H-Lys(Boc)-Leu-Ser(t-Bu)-Gln-Glu(O-t-Bu)-Leu-His(Trt)-
Lys(Boc)-Leu-Gln-Thr(t-Bu)- -Tyr(t-Bu)-Pro-Arg(Mtr)-Thr(t-Bu)-Asp(O-t-Bu)-Val-Leu-
Gly-Ala-Gly-Thr(t-Bu)-ProNH2.
 Obtained: 0.41 g (66%) of crude product. Rf= 0.60 (silica gel plates, eluent butanol-acetic acid-water 4:1:1)
MS (FAB, 8-10 kV, 2mA) m/z = 4577 (M2H)⁺

### Step (b)

H H
CO——CH2—CH2— C=C —CH2
Ser-Asn-Leu-Ser-Thr-NH-CH-CO-Val-Leu-Gly-Lys-Leu-Ser-Gln
-Glu-Leu-His-Lys-Leu-Gln-Thr-Tyr-Pro-Arg-Thr-Asp-Val-Gly-Ala-
-Gly-Thr-ProNH2.4TFA.

The title compound was prepared as described in Example 1(b) starting from 0.41 g of 
H H
CO------------CH2-----CH2 —— C=C———————CH2
Ser(t-Bu)-Asn-Leu-Ser(t-Bu)-Thr(t-Bu)-NH-CH-CO-Val-Leu
-Gly-Lys(Boc)-Leu-Ser(t-Bu)-Gln-Glu(O-t-Bu)-Leu-His(Trt)-
-Lys(Boc)-Leu-Gln-Thr(t-Bu)-Tyr(t-Bu)-Pro-Arg(Mtr)-
-Thr(t-Bu)-Asp(O-t-Bu)-Val-Gly-Ala-Gly-Thr(t-Bu)-Pro-
-NH2.
 The crude product was purified as in Example 1(b), but utilizing the gradient 45% to 90% B in 40 min. Obtained: 10.5 mg of the title compound as a liophyle.
MS(FAB, 8-10 kV, 2mA) m/z = 3362 (M2H)⁺

| **Aminoacid analysis** | | | | | |
|---|---|---|---|---|---|
| Asp | 2.11(2) | Thr | 4.01(4) | Lys | 2.11(2) |
| Glu | 3.40 (3) | Ala | 1.18 (1) | | |
| Ser | 3.04 (3) | Pro | 2.30 (2) | | |
| Gly | 3.29 (3) | Tyr | 1.15 (1) | | |
| His | 1.19 (1) | Val | 2 | | |
| Arg | 1.20 (1) | Leu | 5.29 (5) | | |

### EXAMPLE 3

### Step (a)

H
CO------------CH2-----CH2——C=C————————CH2
H
Ser(t-Bu)-Asn-Leu-Ser(t-Bu)-Thr(t-Bu)-NH-CH-CO-Val-Leu
-Gly-Lys(Boc)-Leu-Ser(t-Bu)-Gln-Glu(O-t-Bu)-Leu-His(Trt)-
-Lys(Boc)-Leu-Gln-Thr(t-Bu)-Tyr(t-Bu)-Pro-Arg(Mtr)-Thr(t-Bu)-
-Asp(O-t-Bu)-Val-Gly-Ala-Gly-Thr(t-Bu)-Pro-NH2

H
CO------------CH2-----CH2 —— C=C---------CH2
H
0.092 g of Ser(t-Bu)-Asn-Leu-Ser(t-Bu)-Thr(t-Bu)-NH-CH-CO-Val-Leu-
-Gly-OH and 0.287 g of H-Lys(Boc)-Leu-Ser(t-Bu)-Gln-Glu(O-t-Bu)-
Leu-His(Trt)-Lys(Boc)-Leu-Gln-Thr(t-Bu)-Tyr(t-Bu)-
-Pro-Arg(Mtr)-Thr(t-Bu)-Asp(O-t-Bu)-Val-Gly-Ala-Gly-
-Thr(t-Bu)-Pro-NH2

(as obtained in Preparation 21) were condensed as described in Example 1(a) affording 0.38 g of the crude title compound, Rf= 0.58 (silica gel plates, eluent butanol-acetic acid-water 4:1:1).
MS (FAB, 8-10 kV, 2mA) m/z = 4577 (M2H)⁺

### Step (b)

H
CO——CH2——CH2—C=C——CH2
H
Ser-Asn-Leu-Ser-Thr-NH-CH-CO-Val-Leu-Val-Gly-Lys-Leu-Ser-Gln
-Glu--Leu-His-Lys-Leu-Gln-Thr-Tyr-Pro-Arg-Thr-Asp-Val-Gly-Ala-Gly-
-Thr-Pro-NH2.4TFA

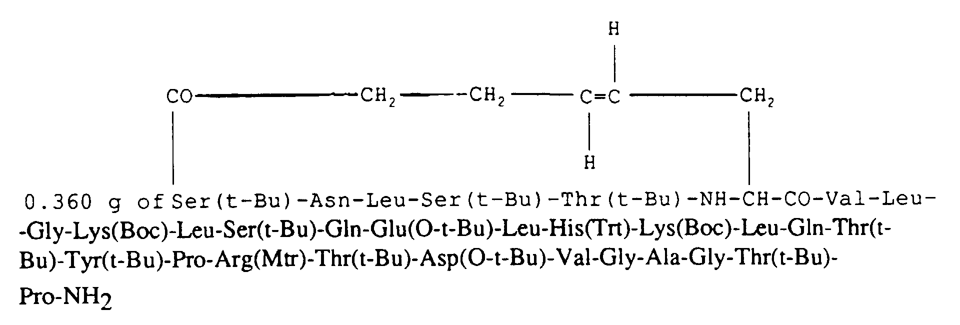
CO——CH2——CH2——C=C——CH2
H (above the C=C), H (below the C=C)
0.360 g of Ser(t-Bu)-Asn-Leu-Ser(t-Bu)-Thr(t-Bu)-NH-CH-CO-Val-Leu-
-Gly-Lys(Boc)-Leu-Ser(t-Bu)-Gln-Glu(O-t-Bu)-Leu-His(Trt)-Lys(Boc)-Leu-Gln-Thr(t-
Bu)-Tyr(t-Bu)-Pro-Arg(Mtr)-Thr(t-Bu)-Asp(O-t-Bu)-Val-Gly-Ala-Gly-Thr(t-Bu)-
Pro-NH2

were treated as described in Example 1(b), affording 0.232 g of the crude title compound. This was purified by HPLC using RP-18 as stationary phase, and a mixture of phosphate buffer (A) and organic phase (B) (stepwise gradient from 10% to 35% B). Phosphate buffer and organic phase (acetonitrile-methanol 4:1) were prepared as described in J. Chromatog., 536, 131-135 (1991). The collected fractions were lyophilized and the residue was further purified by HPLC [RP-18, eluent A=0.05% TFA in water; eluent B=0.05% TFA in water-acetonitrile 3:7; gradient: 0% B for 20 min, then from 0 to 90% B in 15 min). The collected fractions were lyophilized to afford 13 mg of the title compound.
MS(FAB, 8-10 kV, 2mA) m/z = 3362 (M2H)⁺

| **Aminoacid analysis** | | | | | |
|---|---|---|---|---|---|
| Asp | 1.91(2) | Thr | 3.76 (4) | Lys | 2.01(2) |
| Glu | 3.01 (3) | Ala | 1.22 (1) | | |
| Ser | 2.84 (3) | Pro | 2.14 (2) | | |
| Gly | 3.13 (3) | Tyr | 1.22(1) | | |
| His | 1.11(1) | Val | 2 | | |
| Arg | 1.10(1) | Leu | 5.11(5) | | |

### Example 4

### Step (a)

CO------------CH2-----CH2——C≡C——-----CH2
Ser(t-Bu)-Asn-Leu-Ser(t-Bu)-Thr(t-Bu)-NH-CH-CO-Val-Leu
-Gly-Lys(Boc)-Leu-Ser(t-Bu)-Gln-Glu(O-t-Bu)-Leu-His(Trt)-Lys(Boc)-Leu-Gln-
Thr(t-Bu)-Tyr(t-Bu)-Pro-Arg(Mtr)-Thr(t-Bu)-Ala-Val-Gly-Ala-Gly-Thr(t-Bu)-Pro-
NH2

CO------------CH2-----CH2--- C≡C-----------CH2
46 mg of Ser(t-Bu)-Asn-Leu-Ser(t-Bu)-Thr(t-Bu)-NH-CH-CO-Val-Leu- OH
and 141 mg of H-Lys(Boc)-Leu-Ser(t-Bu)-Gln-Glu(O-t-Bu)-Leu-His(Trt)-Lys(Boc)-Leu-
Gln-Thr(t-Bu)-Tyr(t-Bu)-Pro-Arg(Mtr)-Thr(t-Bu)-Ala-Val-Gly-Ala-Gly-Thr(t-Bu)-Pro-
NH2

were treated as described in Example 1, Step (a). Obtained 182 mg (97%), Rf = 0.58 (silica gel plates, eluent butanol-acetic acid-water 4:1:1).

### Step (b)

CO——CH2——CH2—C≡C——CH2
Ser-Asn-Leu-Ser-Thr-NH-CH-CO-Val-Leu-Gly-Lys-Leu-Ser-Gln-

-Glu-Leu-His-Lys-Leu-Gln-Thr-Tyr-Pro-Arg-Thr-Ala-Val-Gly-Ala-Gly-Thr-Pro-
NH2·4TFA

CO-------------CH2-----CH2--- C≡C ---------CH2

175 mg of Ser(t-Bu)-Asn-Leu-Ser(t-Bu)-Thr(t-Bu)-NH-CH-CO-Val-Leu-
-Gly-Lys(Boc)-Leu-Ser-(t-Bu)-Gln-Glu(O-t-Bu)-Leu-His(Trt)-Lys-(Boc)-Leu-Gln-Thr(t-
Bu)-Tyr(t-Bu)-Pro-Arg(Mtr)-Thr(t-Bu)-Ala-Val-Gly-Ala-Gly-Thr(t-Bu)-Pro-NH2

treated as described in Preparation 1, Step (b), obtaining 141 mg of crude product. This was purified as described in Preparation 1, Step (b) but utilizing the gradient 30% to 90% B in 45 min. Obtained: 24.1 mg of the title compound as a lyophile, Rf = 0.30 (silica gel plates, eluent chloroform-methanol-32% ammonia 10:7:3).
MS (FAB, 8-10 kV, 2 mA) m/z = 3315 (MH⁺).

| **Aminoacid analysis** | | | | | | | | |
|---|---|---|---|---|---|---|---|---|
| Asp | 0.96 | (1) | Thr | 3.64 | (4) | Lys | 2.23 | (2) |
| Glu | 3.22 | (3) | Ala | 2.11 | (2) | | | |
| Ser | 2.93 | (3) | Pro | 2.18 | (2) | | | |
| Gly | 3.16 | (3) | Tyr | 1.22 | (1) | | | |
| His | 1.11 | (1) | Val | 2 | | | | |
| Arg | 1.07 | (1) | Leu | 5.22 | (5) | | | |

### Biological Results

The hypocalcaemic activity of compounds (I) can be determined as reported for example in J. Endocrinology, 33, 469 (1965).

Male Sprague Dawley rats, about 4 weeks old, were kept under fasting conditions for 24 hours. The compounds were dissolved with BSA buffer (0.1% bovine serum albumin on 0.1M acetate buffer pH 4.5) and injected by the i.v. route into the lateral tail vein at doses of 3-10-30-100 ng/Kg. One hour after the dosing blood was sampled from the tail or neck vessels, then the serum samples obtained.

The serum was diluted with 0.1% lanthanium chloride solution and the concentration of total calcium was determined by Atomic Absorption Spectrophotometry.

Results obtained with the compounds of Examples 1-4 are reported in table I.

**TABLE 1.**

| **HYPOCALCAEMIC ACTIVITY OF COMPOUNDS (I)** | | | | |
|---|---|---|---|---|
| Total serum calcium (mg/100 ml); mean + S.D. | | | | |
| Dose (ng/kg) | | | | |
| Compound of Example No. | 3 | 10 | 30 | 100 |
| VEHICLE 10.49 + 0.6 | | | | |
| 1 | 10.16±0.8 | 9.16±0.6 | 7.86±0.5 | 7.61±0.4 |
| 2 | 10.36±0.4 | 9.26±0.5 | 8.07±0.3 | 7.36±0.3 |
| 3 | 8.88±0.8 | 8.59±0.7 | 6.95±0.4 | 6.87±0.4 |
| 4 | 10.09±0.1 | 8.85±0.25 | 7.92±0.15 | 7.66±0.18 |

The following illustrate typical formulations according to the present invention.

### Formulations for nasal, sublingual, buccal, rectal or vaginal administration

### Examples 1-3

| | Example No. | | |
|---|---|---|---|
| | 1 | 2 | 3 |
| Compound of Example 1 (µg) | 300 | 300 | 300 |
| Ammonium glycyrrhizinate (g) | 2 | - | 2 |
| Citric acid (mg) | 37 | 37 | 37 |
| Sodium citrate dihydrate | 463 | 463 | 463 |
| Sodium chloride (mg) | 600 | 600 | 600 |
| Benzyl alcohol (g) | - | 1 | 2 |
| Distilled water | | q.s. to 100 ml | |
| 1N NaOH | | q.s. to pH 6 | |

The formulations of Examples 1 to 3 are prepared by mixing together the ammonium glycyrrhizinate (Examples 1 and 3), citric acid, sodium citrate dihydrate, sodium chloride, distilled water and sodium hydroxide in a water bath regulated at a temperature of about 70°C. To the resulting solution cooled to room temperature, benzyl alcohol (Examples 2 and 3) and calcitonin is then added.

### Examples 4-10

The following compositions are prepared according to the method described in Examples 1 to 3.

| | Example No. | | | | | | |
|---|---|---|---|---|---|---|---|
| | 4 | 5 | 6 | 7 | 8 | 9 | 10 |
| Compound of Example 1 (µg) | 3690 | 3690 | 3690 | 7380 | 7380 | 7380 | 7380 |
| Ammonium glycyrrhizinate (g) | 0.5 | - | 5 | - | 1 | 2 | 5 |
| Citric acid (mg) | 37 | 37 | 37 | 37 | 37 | 37 | 37 |
| Sodium citrate dihydrate (mg) | 463 | 463 | 463 | 463 | 463 | 463 | 463 |
| Sodium chloride (mg) | 600 | 600 | 600 | 600 | 600 | 600 | 600 |
| Benzyl alcohol (g) | 2 | 2 | - | 2 | 2 | - | 2 |
| Distilled water | q.s. to 100 ml | | | | | | |
| 1N NaOH | q.s. to pH 6 | | | | | | |

### Examples 11-14

| | Example No. | | | |
|---|---|---|---|---|
| | 11 | 12 | 13 | 14 |
| Compound of Example 1 (µg) | 3690 | 3690 | 7380 | 7380 |
| Ammonium glycyrrhizinate (g) | 2 | 2 | 2 | 2 |
| Citric acid (mg) | 37 | 37 | 37 | 37 |
| Sodium citrate dihydrate (mg) | 463 | 463 | 463 | 463 |
| Sodium chloride (mg) | 600 | 600 | 600 | 600 |
| Benzyl alcohol (g) | - | 1 | - | 1 |
| Methyl p-hydroxybenzoate (mg) | 130 | 130 | 130 | 130 |
| Propyl p-hydroxybenzoate (mg) | 20 | 20 | 20 | 20 |
| Distilled water | q.s. to 100 ml | | | |
| IN NaOH | q.s. to pH 6 | | | |

The formulations of Examples 11 to 14 are prepared by mixing together the ammonium glycyrrhizinate, citric acid, sodium citrate dihydrate, sodium chloride, methyl p-hydroxybenzoate, propyl p-hydroxybenzoate, distilled water and sodium hydroxide in a water bath regulated at a temperature of about 70°C. To the resulting solution cooled to room temperature, benzyl alcohol (Examples 12 and 14) and calcitonin is then added.

### Powder for nasal administration

### Examples 15 and 16

| | Example No. | |
|---|---|---|
| | 15 | 16 |
| Compound of Example 1 (mg) | 3.69 | 7.38 |
| Ammonium glycyrrhizinate (g) | 2.0 | 2.0 |
| Benzyl alcohol (g) | 2.0 | - |
| Lactose q.s. to (g) | 25.0 | 25.0 |

The formulations of Examples 15 and 16 are prepared by wetting the lactose with benzyl alcohol (Example 1) and with an aqueous solution of calcitonin and drying under vacuum. The dried powder is mixed with ammonium glycyrrhizinate and the final mixture is placed into hard gelatine capsules (25 mg each capsule).

The powder is administered, after having pierced the capsules, using a nasal insufflator.

### Sublingual tablets

### Examples 17 and 18

| | Example No. | |
|---|---|---|
| | 17 | 18 |
| Compound of Example 1 (mg) | 7.7 | 15.4 |
| Ammonium glycyrrhizinate (g) | 4.0 | 4.0 |
| Benzyl alcohol (g) | - | 4.0 |
| Sucrose (g) | 35.0 | 35.0 |
| Mannitol (g) | 35.0 | 35.0 |
| Polyethylene glycol 6000 (g) | 10.0 | 10.0 |
| Lactose q.s. to (g) | 120.0 | 120.0 |

The formulations of Examples 17 and 18 are prepared by mixing together the sucrose, the mannitol and the lactose. The resulting mixture is wetted with benzyl alcohol (Example 18) and with an aqueous solution of calcitonin, granulated through a stainless steel screen and dried under vacuum. The dried granules are mixed with polyethylene glycol and ammonium glycyrrhizinate and then compressed into tablets of 120 mg each.

### Buccal tablets

### Examples 19 and 20

| | Example No. | |
|---|---|---|
| | 19 | 20 |
| Compound of Example 1 (mg) | 7.7 | 15.4 |
| Ammonium glycyrrhizinate (g) | 4.0 | 4.0 |
| Sucrose (g) | 30.0 | 30.0 |
| Mannitol (g) | 35.0 | 35.0 |
| Polyethylene glycol 6000 (g) | 15.0 | 15.0 |
| Carbopol 934 (g) | 15.0 | 15.0 |
| Lactose q.s. to (g) | 150.0 | 150.0 |

The formulations of Examples 19 and 20 are prepared by mixing together the sucrose, the mannitol and the lactose. The resulting mixture is wetted with an aqueous solution of calcitonin, granulated through a stainless steel screen and dried under vacuum. The dried granules are mixed with ammonium glycyrrhizinate, Carbopol and polyethylene glycol and then compressed into tablets of 150 mg each.

### Oral tablets for colonic delivery

### Examples 21 and 22

| | Example No. | |
|---|---|---|
| | 21 | 22 |
| Compound of Example 1 (mg) | 15.4 | 30.8 |
| Ammonium glycyrrhizinate (g) | 6.0 | 6.0 |
| Benzyl alcohol (g) | 4.0 | 4.0 |
| Pregelatinized starch (g) | 80.0 | 80.0 |
| Magnesium stearate (g) | 2.0 | 2.0 |
| Lactose q.s. to (g) | 210.0 | 210.0 |
| Eudragit S (g) | 20.0 | 20.0 |
| Polyethylene glycol 6000 (g) | 2.0 | 2.0 |

The formulations of Examples 21 and 22 are prepared by mixing together the pregelatinized starch and the lactose. The resulting mixture is wetted with benzyl alcohol and with an aqueous solution of calcitonin, granulated through a stainless steel screen and dried under vacuum. The dried granules are mixed with ammonium glycyrrhizinate and magnesium stearate and then compressed into tablets of 210 mg each.

The tablets are coated with an aqueous suspension of polyethylene glycol and Eudragit, to a final weight of 232 mg/tablet.

### Dosage form for vaginal administration

### Examples 23 and 24

| | Example No. | |
|---|---|---|
| | 23 | 24 |
| Compound of Example 1 (mg) | 15.4 | 30.8 |
| Ammonium glycyrrhizinate (g) | 8.0 | 8.0 |
| Benzyl alcohol (g) | - | 8.0 |
| Corn starch (g) | 180.0 | 180.0 |
| Adipic acid (g) | 140.0 | 140.0 |
| Sodium bicarbonate (g) | 110.0 | 110.0 |
| Magnesium stearate (g) | 20.0 | 20.0 |
| Lactose q.s. to (g) | 1600.0 | 1600.0 |

The formulations of Examples 23 and 24 are prepared by mixing together the ammonium glycyrrhizinate, the corn starch, the adipic acid and the lactose. The resulting mixture is wetted with benzyl alcohol (Example 24) and with an aqueous solution of calcitonin, granulated through a stainless steel screen and dried under vacuum. The dried granules are mixed with sodium bicarbonate and magnesium stearate and then compressed into tablets of 1.6 g each.

### Dosage form for rectal administration

### Example 25

| | |
|---|---|
| Compound of Example 1 | 15.4 |
| Ammonium glycyrrhizinate (g) | 6.0 |
| Benzyl alcohol (g) | 4.0 |
| Distilled water (g) | 100.0 |
| Hard fat q.s. to (g) | 1500.0 |

The formulation of Example 25 is prepared by mixing together the ammonium glycyrrihizinate, benzyl alcohol and distilled water in a water bath regulated at a temperature of about 70°C. The solution is cooled to about 40°C, the calcitonin is dissolved and then the resulting solution is incorporated into hard fat melted at about 40°C. The final mixture is poured into suppository moulds and cooled to room temperature, thus obtaining suppositories of 1.5 g each.

### Formulations for vaginal or rectal administration

### Examples 26 - 28

| | Example No. | | |
|---|---|---|---|
| | 26 | 27 | 28 |
| Compound of Example 1 (mg) | 7.4 | 14.8 | 29.6 |
| Polyethylene glycol 600 (g) | 550.0 | 550.0 | 550.0 |
| Gelucire 44/14 (g) | 400.0 | 400.0 | 400.0 |
| Distilled water (g) | 42.1 | 42.1 | 42.1 |
| Ammonium glycyrrhizinate (g) | - | 5.0 | 5.0 |
| Benzyl alcohol (g) | 2.0 | - | 2.0 |
| Sodium chloride (mg) | 300.0 | 300.0 | 300.0 |
| Sodium citrate dihydrate (mg) | 231.5 | 231.5 | 231.5 |
| Sodium hydroxide (mg) | 350.0 | 350.0 | 350.0 |
| Citric acid (mg) | 18.5 | 18.5 | 18.5 |

The formulations of Examples 26 to 28 are prepared by mixing together the ammonium glycyrrhizinate (Examples 27 and 28), citric acid, sodium citrate dihydrate, sodium chloride, sodium hydroxide, distilled water and calcitonin in a water bath regulated at a temperature of about 70°C. The resulting solution, cooled to about 55°C, is incorporated into a mixture of Gelucire, polyethylene glycol and benzyl alcohol (Examples 26 and 28) heated to about 55°C. The final mixture, cooled to about 30°C, is filled into soft gelatin capsules (1 g each capsule).

### Examples 29 - 31

| | Example No. | | |
|---|---|---|---|
| | 29 | 30 | 31 |
| Compound of Example 1 (mg) | 7.4 | 14.8 | 29.6 |
| Polyethylene glycol 600 (g) | 400.0 | 400.0 | 400.0 |
| Witepsol S55 (g) | 450.0 | 450.0 | 450.0 |
| Miglyol 812 (g) | 100.0 | 100.0 | 100.0 |
| Distilled water (g) | 42.1 | 42.1 | 42.1 |
| Ammonium glycyrrhizinate (g) | - | 5.0 | 5.0 |
| Benzyl alcohol (g) | 2.0 | - | 2.0 |
| Sodium chloride (mg) | 300.0 | 300.0 | 300.0 |
| Sodium citrate dihydrate (mg) | 231.5 | 231.5 | 231.5 |
| Sodium hydroxide (mg) | 350.0 | 350.0 | 350.0 |
| Citric acid (mg) | 18.5 | 18.5 | 18.5 |

The formulations of Examples 29 to 31 are prepared by mixing together the ammonium glycyrrhizinate (Examples 30 and 31), citric acid, sodium citrate dihydrate, sodium chloride, sodium hydroxide, distilled water and calcitonin in a water bath regulated at a temperature of about 70°C. The resulting solution, cooled to about 55°C, is incorporated into a mixture of Witepsol, Miglyol, polyethylene glycol and benzyl alcohol (Examples 29 and 31) heated to about 55°C. The final mixture, cooled to about 30°C, is filled into soft gelatin capsules (1 g each capsule).

### Dosage form for transdermal administration

### Example 32

| | |
|---|---|
| Compound of Example 1 (mg) | 6 |
| Ammonium glycyrrhizinate (g) | 2 |
| Benzyl alcohol (g) | 2 |
| Carbopol 934 (g) | 2 |
| Citric acid (mg) | 37 |
| Sodium citrate dihydrate (mg) | 463 |
| Sodium chloride (mg) | 600 |
| Distilled water | q.s.to 100 g |
| 1N NaOH | q.s. to pH 6 |

The formulation of Example 32 is prepared by mixing together the ammonium glycyrrhizinate, citric acid, sodium citrate dihydrate, sodium chloride, Carbopol 934, sodium hydroxide and part of distilled water in a water bath regulated at a temperature of about 70°C. To the resulting gel, cooled to room temperature, a solution of calcitonin and benzyl alcohol in the remaining part of distilled water, is then added. The final gel is filled into patches of 500 mg each.

The compounds of Examples 2-4 may also be formulated as described in Examples 1-32.

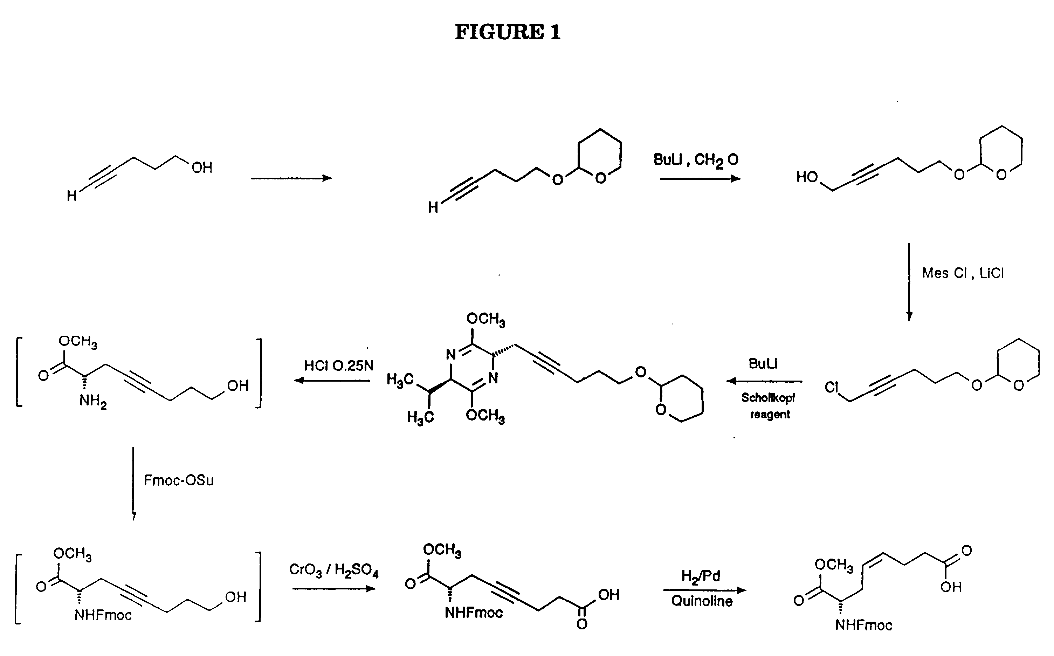

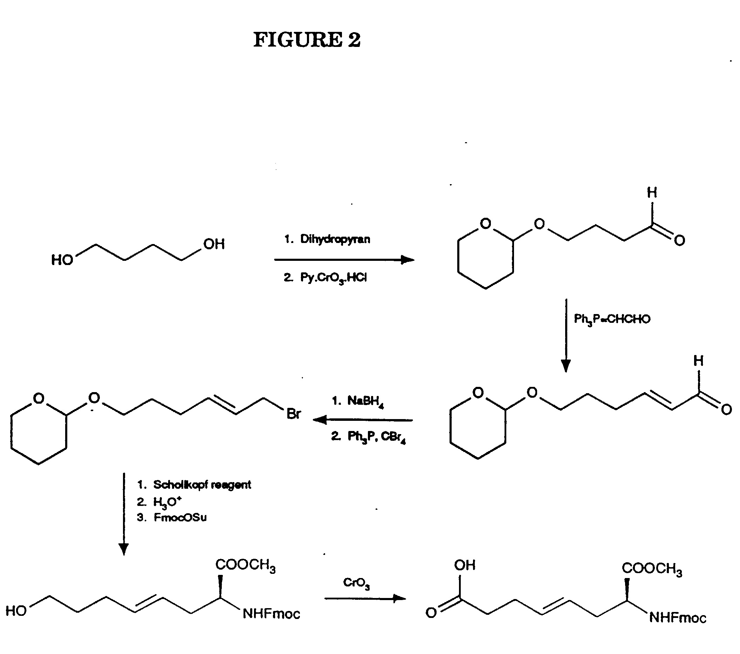

## Claims

1. A compound of formula (I): 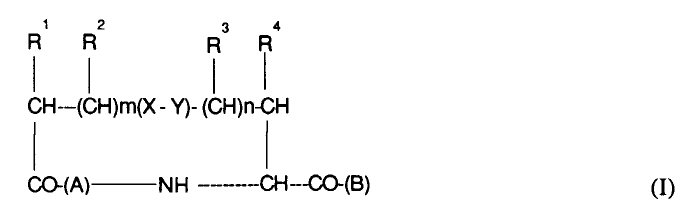 wherein:
(X-Y) represents CH=CH or C≡C;
A represents fragments 2-6 of a naturally occurring calcitonin, or a peptide residue: -A2-A3-Leu-Ser-Thr wherein A₂ represents Ser, Gly or Ala and A₃ represents Asn or Ser;
B represents fragments 8-32 of a naturally occurring calcitonin, or has the formula 
-Val-Leu-Gly-Lys-Leu-Ser-Gln-Glu-Leu-His-Lys-C-Pro-Arg-Thr-D-Gly-E-
Gly-Thr-Pro-NH2
 in which
C represents -Leu-Gln-Thr-Tyr; Gln-Thr-Tyr; or a bond;
D represents -Asn-Thr; -Asp-Val; -Ala-Thr or -Ala-Val; and
E represents Ser or Ala
m and n each independently represent 0, 1 or 2; and
R¹, R², R³, and R⁴ independently represent hydrogen or a C₁₋₄ alkyl group.

2. A compound according to claim 1 wherein (A) represents fragments 2-6 of naturally occurring pig, ox, sheep, human, rat, eel, salmon or chicken calcitonins.

3. A compound according to claim 1 or claim 2 wherein (B) represents fragments 8-32 corresponding to those found in naturally occurring calcitonins selected from:

-Met-Leu-Gly-Thr-Tyr-Thr-Gln-Asp-Phe-Asn-Lys-Phe-His-Thr-Phe-Pro-Gln-Thr-Ala-Ile-
Gly-Val-Gly-Ala-Pro-NH2; (human)

-Met-Leu-Gly-Thr-Tyr-Thr-Gln-Asp-Leu-Asn-Lys-Phe-His-Thr-Phe-Pro-Gln-Thr-Ser-Ile-
Gly-Val-Gly-Ala-Pro-NH2; (rat)

-Val-Leu-Gly-Lys-Leu-Ser-Gln-Glu-Leu-His-Lys-Leu-Gln-Thr-Tyr-Pro-Arg-Thr-Asn-Thr-
Gly-Ser-Gly-Thr-Pro-NH2; (salmon)

-Val-Leu-Gly-Lys-Leu-Ser-Gln-Glu-Leu-His-Lys-Leu-Gln-Thr-Tyr-Pro-Arg-Thr-Asp-Val-
Gly-Ala-Gly-Thr-Pro-NH2; (eel, chicken)

-Val-Leu-Ser-Ala-Tyr-Trp-Lys-Asp-Leu-Asn-Asn-Tyr-His-Arg-Phe-Ser-Gly-Met-Gly-Phe-
Gly-Pro-Glu-Thr-Pro-NH2; (ox)

-Val-Leu-Ser-Ala-Tyr-Trp-Arg-Asn-Leu-Asn-Asn-Phe-His-Arg-Phe-Ser-Gly-Met-Gly-Phe-
Gly-Pro-Glu-Thr-Pro-NH2; (pig) and

-Val-Leu-Ser-Ala-Tyr-Trp-Lys-Asp-Leu-Asn-Asn-Tyr-His-Arg-Tyr-Ser-Gly-Met-Gly-Phe-
Gly-Pro-Glu-Thr-Pro-NH2; (sheep).

4. A compound according to any claims 1 to 3 wherein m represents 1 and n represents 0.

5. A compound according to any of claims 1 to 4 wherein R¹-R⁴ each represent hydrogen.

6. A compound according to any of claims 1 to 5 wherein (X-Y) represents C≡C.

7. A process for the preparation of a compound of formula (I) as defined in any of claims 1 to 6 which comprises subjecting amino-acids, peptides or combinations thereof to condensation reaction(s) to form an amino-acid sequence in the order of formula (I) wherein active groups not participating in the condensation reaction are protected and at any appropriate stage of the reaction subjecting to cyclisation the structural units containing a peptide residue of formula (II) : 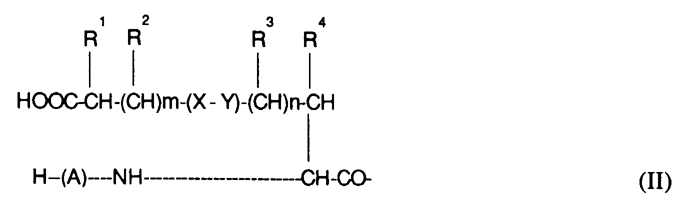 (wherein A, X-Y, m, n and R¹-R⁴ are as defined above)
and finally removing any protecting groups to give a compound of formula (I).

8. The compound of formula (IIIa) 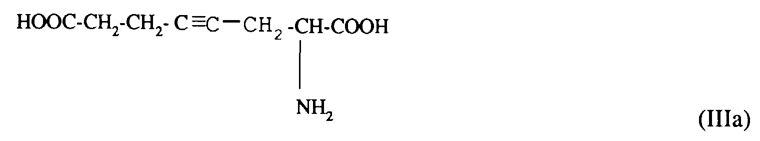 or a protected derivative thereof.

9. Use of a compound of formula (I) as defined in any of claims 1 to 6 in the manufacture of a medicament for the treatment of a condition selected from osteoporosis, Paget's disease, Sudeck's disease and hypercalcaemic conditions..

10. A pharmaceutical composition comprising a compound of formula (I) as defined in any of claims 1 to 6 or a pharmaceutically acceptable salt thereof, and a pharmaceutically acceptable carrier.

## Patentansprüche

1. Verbindung der Formel (I) : 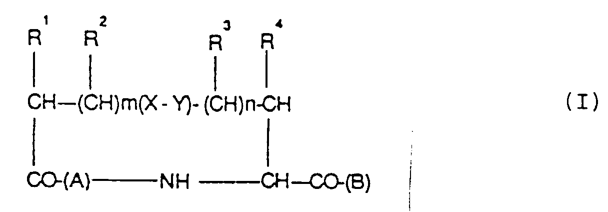 in der
(X-Y) die Gruppe CH=CH oder C≡C darstellt;
A die Fragmente 2 bis 6 eines natürlich vorkommenden Calcitonins darstellt oder einen Peptidrest: -A2-A3-Leu-Ser-Thr, wobei A₂ Ser, Gly oder Ala bedeutet und A₃ Asn oder Ser darstellt;
B die Fragmente 8-32 eines natürlich vorkommenden Calcitonins darstellt oder die Formel hat: 
-Val-Leu-Gly-Lys-Leu-Ser-Gln-Glu-Leu-His-Lys-C-
Pro-Arg-Thr-D-Gly-E-Gly-Thr-Pro-NH2,
 in der
C -Leu-Gln-Thr-Tyr, Gln-Thr-Tyr oder eine Bindung darstellt
D -Asn-Thr, -Asp-Val, -Ala-Thr oder -Ala-Val bedeutet und
E Ser oder Ala darstellt,
m und n jeweils unabhängig voneinander 0, 1 oder 2 bedeuten; und
R¹, R², R³ und R⁴ unabhängig voneinander ein Wasserstoffatom oder einen C₁₋₄-Alkylrest bedeuten.

2. Verbindung nach Anspruch 1, wobei (A) die Fragmente 2 bis 6 der natürlich vorkommenden Calcitonine von Schwein, Ochse, Schaf, Mensch, Ratte, Aal, Lachs oder Huhn darstellt.

3. Verbindung nach Anspruch 1 oder 2, wobei (B) die Fragmente 8 bis 32 bedeutet, die denen entsprechen, die in natürlich vorkommenden Calcitoninen auftreten, ausgewählt aus:

-Met-Leu-Gly-Thr-Tyr-Thr-Gln-Asp-Phe-Asn-Lys-Phe-His-
Thr-Phe-Pro-Gln-Thr-Ala-Ile-Gly-Val-Gly-Ala-Pro-NH2;
(Mensch)

-Met-Leu-Gly-Thr-Tyr-Thr-Gln-Asp-Leu-Asn-Lys-Phe-His-
Thr-Phe-Pro-Gln-Thr-Ser-Ile-Gly-Val-Gly-Ala-Pro-NH2;
(Ratte)

-Val-Leu-Gly-Lys-Leu-Ser-Gln-Glu-Leu-His-Lys-Leu-Gln-
Thr-Tyr-Pro-Arg-Thr-Asn-Thr-Gly-Ser-Gly-Thr-Pro-NH2;
(Lachs)

-Val-Leu-Gly-Lys-Leu-Ser-Gln-Glu-Leu-His-Lys-Leu-Gln-
Thr-Tyr-Pro-Arg-Thr-Asp-Val-Gly-Ala-Gly-Thr-Pro-NH2,
(Aal, Huhn)

-Val-Leu-Ser-Ala-Tyr-Trp-Lys-Asp-Leu-Asn-Asn-Tyr-His-
Arg-Phe-Ser-Gly-Met-Gly-Phe-Gly-Pro-Glu-Thr-Pro-NH2;
(Ochse)

-Val-Leu-Ser-Ala-Tyr-Trp-Arg-Asn-Leu-Asn-Asn-Phe-His-
Arg-Phe-Ser-Gly-Met-Gly-Phe-Gly-Pro-Glu-Thr-Pro-NH2;
(Schwein) und

-Val-Leu-Ser-Ala-Tyr-Trp-Lys-Asp-Leu-Asn-Asn-Tyr-His-
Arg-Tyr-Ser-Gly-Met-Gly-Phe-Gly-Pro-Glu-Thr-Pro-NH2;
(Schaf).

4. Verbindung nach einem der Ansprüche 1 bis 3, wobei m den Wert 1 und n den Wert 0 hat.

5. Verbindung nach einem der Ansprüche 1 bis 4, wobei R¹ bis R⁴ jeweils Wasserstoffatome bedeuten.

6. Verbindung nach einem der Ansprüche 1 bis 5, wobei (X-Y) C≡C darstellt.

7. Verfahren zur Herstellung einer Verbindung der Formel (I) gemäß der Definition in einem der Ansprüche 1 bis 6, umfassend die Durchführung einer (von) Kondensationsreaktion(en) mit Aminosäuren, Peptiden oder Kombinationen davon zur Erzeugung einer Aminosäuresequenz in der Reihenfolge der Formel (I), wobei aktive Gruppen, die nicht an der Kondensationsreaktion teilnehmen, geschützt sind, und zu einem geeigneten Zeitpunkt der Umsetzung Cyclisierung der Struktureinheiten, die einen Peptidrest der Formel (II) enthalten: 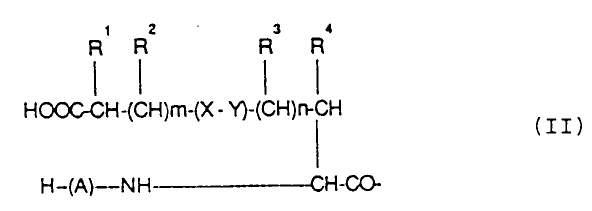 wobei A, X-Y, m, n und R¹ bis R⁴ die vorstehend angegebene Bedeutung haben,
und schließlich Entfernung von Schutzgruppen zur Erzeugung einer Verbindung der Formel (I).

8. Verbindung der Formel (IIIa) 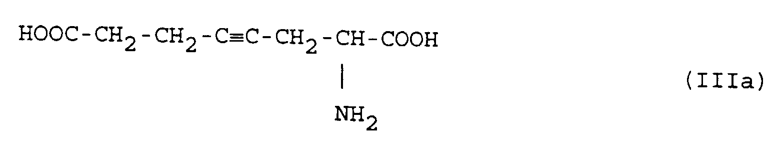 oder eines geschützten Derivats davon.

9. Verwendung einer Verbindung der Formel (I) gemäß der Definition in einem der Ansprüche 1 bis 6 für die Herstellung eines Medikamentes zur Behandlung eines Zustandes, ausgewählt aus Osteoporose, Paget-Krankheit, Sudeck-Krankheit und hypercalcämischen Zuständen.

10. Arzneimittel, umfassend eine Verbindung der Formel (I) gemäß der Definition in einem der Ansprüche 1 bis 6 oder ein pharmazeutisch verträgliches Salz davon und einen pharmazeutisch verträglichen Träger.

## Revendications

1. Composé de formule (I) : 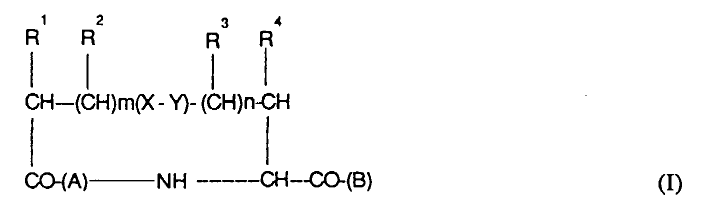 dans laquelle :
(X-Y) représentent un groupe CH=CH ou C≡C ;
A représente les fragments 2-6 d'une calcitonine naturelle, ou un résidu peptidique : -A2-A3-Leu-Ser-Thr dans lequel A₂ représente Ser, Gly ou Ala et A₃ représente Asn ou Ser ;
B représente les fragments 8-32 d'une calcitonine naturelle, ou bien répond à la formule 
-Val-Leu-Gly-Lys-Leu-Ser-Gln-Glu-Leu-His-Lys-C-Pro-Arg-Thr-D-Gly-E-
Gly-Thr-Pro-NH2
 dans laquelle
C représente -Leu-Gln-Thr-Tyr Gln-Thr-Tyr ; ou une liaison ;
D représente -Asn-Thr ; -Asp-Val ; -Ala-Thr ou -Ala-Val ; et
E représente Ser ou Ala
m et n représentent chacun indépendamment 0, 1 ou 2 ; et
R¹, R², R³ et R⁴ représentent indépendamment l'hydrogène ou un groupe alkyle en C₁ à C₄.

2. Composé suivant la revendication 1, dans lequel (A) représente les fragments 2-6 de la calcitonine naturelle de porc, de boeuf, de mouton, d'homme, de rat, d'anguille, de saumon ou de poulet.

3. Composé suivant la revendication 1 ou la revendication 2, dans lequel (B) représente les fragments 8-32 correspondant à ceux présents dans des calcitonines naturelles, choisis entre :

-Met-Leu-Gly-Thr-Tyr-Thr-Gln-Asp-Phe-Asn-Lys-Phe-His-Thr-Phe-Pro-Gln-Thr-Ala-Ile-
Gly-Val-Gly-Ala-Pro-NH2; (homme)

-Met-Leu-Gly-Thr-Tyr-Thr-Gln-Asp-Leu-Asn-Lys-Phe-His-Thr-Phe-Pro-Gln-Thr-Ser-Ile-
Gly-Val-Gly-Ala-Pro-NH2; (rat)

-Val-Leu-Gly-Lys-Leu-Ser-Gln-Glu-Leu-His-Lys-Leu-Gln-Thr-Tyr-Pro-Arg-Thr-Asn-Thr-
Gly-Ser-Gly-Thr-Pro-NH2; (saumon)

-Val-Leu-Gly-Lys-Leu-Ser-Gln-Glu-Leu-His-Lys-Leu-Gln-Thr-Tyr-Pro-Arg-Thr-Asp-Val-
Gly-Ala-Gly-Thr-Pro-NH2; (anguille, poulet)

-Val-Leu-Ser-Ala-Tyr-Trp-Lys-Asp-Leu-Asn-Asn-Tyr-His-Arg-Phe-Ser-Gly-Met-Gly-Phe-
Gly-Pro-Glu-Thr-Pro-NH2; (boeuf)

-Val-Leu-Ser-Ala-Tyr-Trp-Arg-Asn-Leu-Asn-Asn-Phe-His-Arg-Phe-Ser-Gly-Met-Gly-Phe-
Gly-Pro-Glu-Thr-Pro-NH2; (porc) et

-Val-Leu-Ser-Ala-Tyr-Trp-Lys-Asp-Leu-Asn-Asn-Tyr-His-Arg-Tyr-Ser-Gly-Met-Gly-Phe-
Gly-Pro-Glu-Thr-Pro-NH2; (mouton).

4. Composé suivant l'une quelconque des revendications 1 à 3, dans lequel m est égal à 1 et n est égal à 0.

5. Composé suivant l'une quelconque des revendications 1 à 4, dans laquelle R¹ à R⁴ représentent chacun l'hydrogène.

6. Composé suivant l'une quelconque des revendications 1 à 5, dans lequel (X-Y) représente un groupe C≡C.

7. Procédé pour la préparation d'un composé de formule (I) répondant à la définition suivant l'une quelconque des revendications 1 à 6, qui comprend les étapes consistant à soumettre des aminoacides, des peptides ou leurs associations à une ou plusieurs réactions de condensation pour former une séquence d'aminoacides dans l'ordre de la formule (I), les groupes actifs ne participant pas à la réaction de condensation étant protégés, et, à n'importe quel stade approprié de la réaction, à soumettre à une cyclisation les motifs structuraux contenant un résidu peptidique de formule (II) : 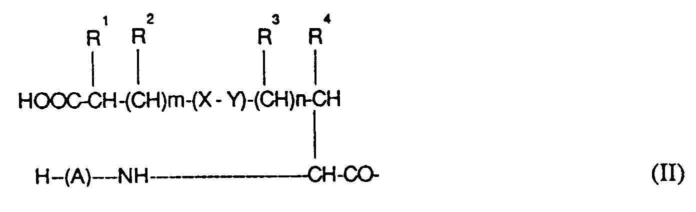 (dans laquelle A, X-Y, m, n et R¹-R⁴ répondent aux définitions précitées)
et, finalement, à éliminer tous les groupes protecteurs, ce qui donne un composé de formule (I).

8. Composé de formule (IIIa) 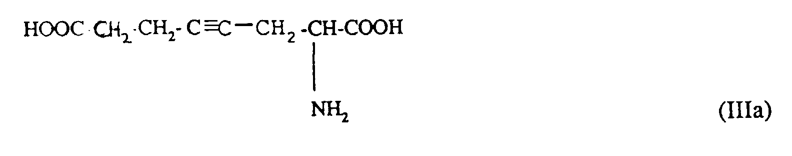 ou un de ses dérivés protégés.

9. Utilisation d'un composé de formule (I) répondant à la définition suivant l'une quelconque des revendications 1 à 6 dans la production d'un médicament destiné au traitement d'une affection faisant partie du groupe consistant en l'ostéoporose, la maladie de Paget, la maladie de Sudeck et des états hypercalcémiques.

10. Composition pharmaceutique comprenant un composé de formule (I) répondant à la définition suivant l'une quelconque des revendications 1 à 6 ou un de ses sels pharmaceutiquement acceptables, et un support pharmaceutiquement acceptable.
